(19) Europäisches Patentamt
European Patent Office
Office européen des brevets

(11) Veröffentlichungsnummer: **0 031 089**
**B1**

(12) # EUROPÄISCHE PATENTSCHRIFT

(45) Veröffentlichungstag der Patentschrift: **15.02.84**

(21) Anmeldenummer: **80107820.5**

(22) Anmeldetag: **11.12.80**

(51) Int. Cl.³: **C 07 C 85/06,** C 07 C 87/50, C 07 C 93/14, C 07 D 295/02, C 07 D 295/04, C 07 C 87/64, C 07 C 97/24, C 07 C 103/28, C 07 C 143/58, C 07 C 143/80

(54) **Verfahren zur Herstellung von N,N'-Diarylalkylendiaminen oder N,N',N''-Triaryldialkylen-triaminen.**

(30) Priorität: **20.12.79 DE 2951400**
**16.09.80 DE 3034879**

(43) Veröffentlichungstag der Anmeldung:
**01.07.81 Patentblatt 81/26**

(45) Bekanntmachung des Hinweises auf die Patenterteilung:
**15.02.84 Patentblatt 84/7**

(84) Benannte Vertragsstaaten:
**CH DE FR GB IT LI**

(56) Entgegenhaltungen:
**DE - A - 2 646 379**
**DE - A - 2 916 991**
**US - A - 2 921 093**

(73) Patentinhaber: **HOECHST AKTIENGESELLSCHAFT, Postfach 80 03 20, D-6230 Frankfurt am Main 80 (DE)**

(72) Erfinder: **Mohr, Reinhard, Dr., Rohrstrasse 34, D-6050 Offenbach/Main (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 031 089 B1

# Verfahren zur Herstellung von N,N'-Diarylalkylendiaminen oder N,N',N''-Triaryldialkylen-triaminen

Die vorliegende Erfindung liegt auf dem Gebiet der Herstellung von Arylaminalkylen-Verbindungen. Sie stellt ein verbessertes Verfahren dar.

1,2-Diphenylamino-alkan-Verbindungen entsprechend dem Typ der später genannten allgemeinen Formel (7) und N,N'-Diphenyl-piperazin Verbindungen des Typs der später stehenden allgemeinen Formel (6) und deren Herstellung sind bekannt. So werden beispielsweise in Chem. Ber. *22*, 1778 (1889) und *40*, 763 (1907) sowie in J. Chem. Soc. (London) *95*, 417 (1909) und *103*, 1609 (1913) Verfahren zur Herstellung solcher Verbindungen beschrieben, in welchen Dibromäthan und gemäss Liebigs Ann. Chem. *471*, 76 (1929) und Chem. Ber. *22*, 2092 (1889) Chloräthanol und gemäss J. Chem. Soc. (London) *121*, 647 (1922) p-Toluolsulfonsäureester von N-$\beta$-Hydroxyäthyl-anilinen oder n-$\beta$-Chloräthylaniline jeweils mit Anilinderivaten umgesetzt werden; die hierbei verwendeten Ausgangsverbindungen sind jedoch teilweise giftige Stoffe, die eine technische Verwertung dieser Herstellungsmethoden verbieten. Des weiteren sind beispielsweise aus Liebigs Ann. Chem. *173*, 131 (1874), aus J. Am. Chem. Soc. *61*, 532 (1939) und J. Gen. Chem. (UdSSR) *24*, 1429 (1954) ebenfalls Verfahren zur Herstellung solcher Verbindungen bekannt, bei denen N-$\beta$-Hydroxyäthyl-anilin-Derivate mit Anilinverbindungen bei hoher Temperatur mit oder ohne Hilfe eines Kondensationsmittels umgesetzt werden.

In der letztgenannten Literaturstelle ist ausserdem die Umsetzung von Monoäthylenglykol mit N,N'-Diphenyläthylendiamin zum N,N'-Diphenylpiperazin mit Aluminiumsilicat als Katalysator beschrieben. Bei diesen bekannten Verfahren arbeitet man bei Temperaturen zwischen 230 und 350°C, ebenso in Lösungsmitteln bei Gegenwart eines Überschusses an Phosphorpentoxid als Kondensationssmittel [J. Am. Chem. Soc. *42*, 1725 (1920)]. Neben diesen Nachteilen, die Verfahren bei sehr hohen Temperaturen und mit grösseren Überschüssen an einem Kondensationsmittel durchzuführen, zeigen sich teilweise nur mässige Ausbeuten unter Lieferung von verunreinigten Produkten.

Mit der vorliegenden Erfindung wurde nunmehr ein Verfahren gefunden, das diese Nachteile vermeidet. Sie betrifft demnach ein verbessertes Verfahren zur Herstellung von N,N'-Diarylalkylen-diaminen und von N,N',N''-Triaryldialkylen-triaminen der allgemeinen Formel (1)

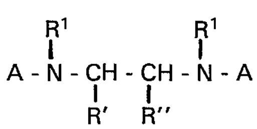

(1)

in welcher

beide A zueinander gleiche oder voneinander verschiedene Bedeutungen haben und jedes einen Arylrest, bevorzugt einen Naphthyl- oder Anthracenyl-Rest, insbesondere bevorzugt einen Phenyl-Rest darstellt, die jeweils substituiert sein können,

R' und R'' zueinander gleiche oder voneinander verschiedene Bedeutungen haben und jedes ein Wasserstoffatom oder eine nieder Alkylgruppe, wie eine Methyl- oder Äthylgruppe, bedeutet,

beide $R^1$ zueinander gleiche oder voneinander verschiedene Bedeutungen haben und jedes ein Wasserstoffatom oder ein gegebenenfalls substituierter Alkyl-, Aralkyl-, Aryl- oder Cycloalkylrest oder eines der $R^1$ eine Gruppe der allgemeinen Formel (2)

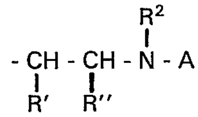

(2)

ist, in welcher

R', R'' und A die obengenannte Bedeutung haben und

$R^2$ ein Wasserstoffatom oder ein gegebenenfalls substituierter Alkyl-, Aralkyl-, Aryl- oder Cycloalkylrest ist,

wobei $R^2$ in Formel (2) die gleiche oder eine davon verschiedene Bedeutung von $R^1$ von Formel (1) und A zu den beiden A in Formel (1) jeweils gleiche oder verschiedene Bedeutungen besitzen können,

oder worin,

beide $R^1$ zusammen vereinigt einen Rest der Formel

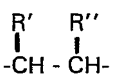 bzw. 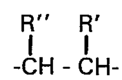

mit R' und R'' der obigen Bedeutung darstellen,

mit welchem verbesserten Verfahren man entsprechende Hydroxyverbindungen mit entsprechenden Aminoverbindungen gemäss den folgenden Verfahrenvarianten umsetzt:

a) ein $\beta$-Hydroxyalkyl-arylamin der allgemeinen Formel (3)

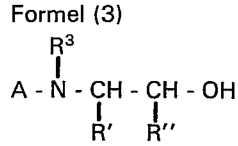

(3)

in welcher A, R' und R'' die obengenannten Bedeutungen haben und $R^3$ für einen gegebenenfalls substituierten Alkyl-, Aralkyl-, Aryl- oder Cycloalkylrest steht, mit einem Amin der allgemeinen Formel (4)

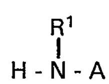

(4)

in welcher A und $R^1$ die obengenannten Bedeutungen haben, zur Verbindung (1), in welcher beide A sowie R' und R'' die obengenannten Bedeutungen haben und das eine $R^1$ ein gegebenenfalls substituierter Alkyl-, Aralkyl-, Aryl- oder Cycloalkylrest ist und das andere $R^1$ ein Wasserstoffatom oder ein gegebenenfalls substituierter Alkyl-, Aralkyl-, Aryl- oder Cycloalkylrest ist, umsetzt oder

b) ein β-Hydroxyalkyl-arylamin der allgemeinen Formel (3a)

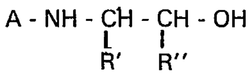 (3a)

in welcher A, R' und R'' die obengenannten Bedeutungen haben, mit einem β-Hydroxyalkyl-arylamin der allgemeinen Formel (3b)

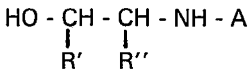 (3b)

in welcher A, R' und R'' die obengenannten Bedeutungen haben, zur Verbindung der Formel (6a) bzw. (6b)

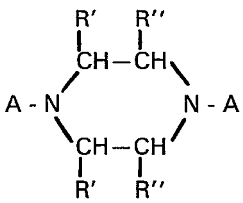

(6a)

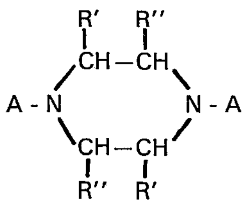

(6b)

in welchen beide A sowie R' und R'' die obengenannten Bedeutungen haben, umsetzt oder

c) eine Alkylenglykol-Verbindung der allgemeinen Formel (5)

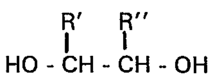 (5)

in welcher R' und R'' die obengenannten Bedeutungen haben, oder dessen Alkylenoxid, wie beispielsweise Propylenoxid oder Butylenoxid, mit einem Amin der allgemeinen Formel (4)

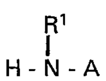 (4)

in welcher $R^1$ und A die obengenannten Bedeutungen haben, oder mit zwei verschiedenen Aminen der Formel (4), in welchen A oder $R^1$ oder beide verschiedene Bedeutungen haben, zur Verbindung (1), in welcher beide A sowie R' und R'' die obengenannten Bedeutungen haben und beide $R^1$, beide gleich oder verschieden voneinander, jeweils für ein Wasserstoffatom oder einen gegebenenfalls substituierten Alkyl-, Aralkyl-, Aryl- oder Cycloalkylrest stehen, umsetzt oder

d) ein Amin der obigen Formel (3), in welcher $R^3$ für eine Gruppe der oben definierten Formel (2) steht und A, R' und R'' die oben genannten Bedeutungen haben, mit einem Amin der obigen Formel (4), in welcher $R^1$ für ein Wasserstoffatom steht und A die obengenannte Bedeutung besitzt, zur Verbindung (1), in welcher beide A sowie $R^1$ und R'' die obengenannten Bedeutungen haben, das eine $R^1$ für die oben definierte Gruppe der Formel (2) steht und das andere $R^1$ ein Wasserstoffatom bedeutet, umsetzt, oder

e) ein Amin der obigen Formel (3), in welcher $R^3$ für eine Gruppe der Formel -CH(R')-CH(R'')-OH steht und A, R' und R'' die obengenannten Bedeutungen haben, mit einem Amin der obigen Formel (4), in welcher A und $R^1$ die obengenannten Bedeutungen haben, zur Verbindung (1), in welcher beide A sowie R', R'' und das eine $R^1$ die obengenannten Bedeutungen haben und das andere $R^1$ für einen Rest der Formel (2) steht, umsetzt oder

f) ein Amin der obigen Formel (3), in welcher A, $R^3$, R' und R'' die obengenannten Bedeutungen haben, mit einem Amin der allgemeinen Formel (4), in welcher A die obengenannte Bedeutung besitzt und $R^1$ für ein Wasserstoffatom steht, zur Verbindung (1), in welcher beide A sowie R', R'' und das eine $R^1$ die hier genannten Bedeutungen haben und das andere $R^1$ für einen Rest der Formel (2) steht, umsetzt oder

g) eine Verbindung der Formel (4), in welcher A die obengenannte Bedeutung besitzt und $R^1$ für ein Wasserstoffatom steht, mit einer Verbindung der Formel (5), in welcher R' und R'' die obengenannten Bedeutungen haben, zur Verbindung der oben definierten Formel (6a) bzw. (6b), in welchen beide A sowie R' und R'' die obengenannten Bedeutungen besitzen, umsetzt oder

h) eine Verbindung der obigen Formel (3), in welcher $R^3$ für eine Gruppe der Formel -CH(R')-CH(R'')-OH steht und A, R' und R'' die obengenannten Bedeutungen haben, mit einer Verbindung der Formel (4), in welcher A die obengenannte Bedeutung besitzt und $R^1$ ein Wasserstoffatom ist, zur Verbindung der oben definierten Formel (6a) bzw. (6b) umsetzt.

Diese Verfahrensweisen sind dadurch gekennzeichnet, dass man die Umsetzung der Verbindungen in Gegenwart von Phosphorsäure oder von phosphoriger Säure in einer Menge von mindestens 0,01 Mol pro Mol Verbindung (3) oder (5) und bei einer Temperatur zwischen 150 und 225°C, vorzugsweise zwischen 170 und 200°C, durchführt.

Die Umsetzung der Amine der allgemeinen Formeln (3) und (4) kann in äquimolaren oder etwa äquimolaren Mengen, insbesondere im Molverhältnis von 1 : 1 bis 1 : 1,1, erfolgen. Sofern Verbindungen der allgemeinen Formel (1) hergestellt werden sollen, in welchen $R^1$ den Rest der Formel (2) bedeutet, werden entsprechende Amine der allgemeinen Formeln (3) und (4) im Molverhältnis von 1 : 2 oder von etwas kleiner als 1 : 2, beispielsweise bis 1 : 2,2, insbesondere im Molverhältnis von 1 : 2,02 bis 1 : 2,2, miteinander umgesetzt. Lässt man ein Amin der allgemeinen Formel (3) mit einem Amin der allgemeinen Formel (4), in welcher $R^1$ ein Wasserstoffatom bedeutet, miteinander reagieren, so kann auch die Umsetzung der Amine (3) und (4) im Molverhältnis von 2 : 1 oder etwas grösser als 2 : 1, beispielsweise bis 2,2 : 1, insbesondere im Molverhältnis von 2,2 : 1 bis 2,02 : 1, erfolgen.

Die Umsetzung der Verbindungen der allgemeinen Formeln (5) und (4) kann in einem Molverhältnis von 1 : 2 oder einem Molverhältnis von etwas kleiner als 1 : 2, beispielsweise bis 1 : 2,2, insbesondere im Molverhältnis von 1 : 2,02 bis 1 : 2,2, oder sofern Verbindungen der allgemeinen Formel (1) hergestellt werden sollen, in welchen beide $R^1$ einen vereinigten Alkylenrest darstellen, somit Piperazinderivate der oben angegebenen und definierten allgemeinen Formeln (6a) und (6b) hergestellt werden sollen, in äquimolarem oder etwa äquimolarem Verhältnis erfolgen; im letztgenannten Falle werden entsprechende Verbindungen der allgemeinen Formeln (5) und (4), in welchen beide $R^1$ Wasserstoff bedeuten, im Molverhältnis von 1 : 1 oder von etwas grösser als 1 : 1, beispielsweise bis 1,1 : 1, insbesondere im Molverhältnis von 1,01 : 1 bis 1,1 : 1, oder aber Verbindungen der oben genannten allgemeinen Formeln (3a) und (3b) in äquimolarem oder etwa äquimolarem Molverhältnis, miteinander umgesetzt.

Die bei der Herstellung der Verbindungen der allgemeinen Formel (1) erfindungsgemäss als saures Kondensationssmittel verwendete Phosphorsäure oder phosphorige Säure wird in der Regel, wie bereits erwähnt, vorteilhaft in katalytischen Mengen von mindestens 0,01 Mol pro Mol Verbindung (3) oder (5) eingesetzt; im allgemeinen wird die Reaktion schon in relativ kurzer Zeit mit 0,01 bis 0,3 Mol Phosphorsäure oder phosphoriger Säure pro Mol der Verbindung (3) oder (5) zu Ende geführt. Die Umsetzung kann jedoch ebenso gut in Gegenwart einer grösseren Menge Phosphorsäure oder phosphoriger Säure, wie beispielsweise in einer mehr als 1-molaren Menge, bezogen auf die eingesetzte Hydroxyalkylamin-Verbindung der Formel (3) oder auf die eingesetzte Glykolverbindung der Formel (5), durchgeführt werden. Im allgemeinen verwendet man jedoch die Phosphorsäure oder phosphorige Säure in einer Menge, die deutlich unter dem äquimolaren Verhältnis, bezogen auf das Hydroxyalkylamin der Formel (3) oder das Glykol der Formel (5), liegt.

Das erfindungsgemässe Verfahren ist zudem deshalb besonders vorteilhaft, weil es die Verwendung auch der handelsüblichen, etwa 80 bis 85%igen Phosphorsäure bzw. handelsüblichen, etwa 50%igen phosphorigen Säure ermöglicht. Diese handelsüblichen Säuren können ohne Schwierigkeiten, beispielsweise für das kontinuierliche Verfahren, unter leichter Dosierung dem Reaktionsansatz zugegeben werden; sie gestatten eine gute Verteilung in der Reaktionsmischung.

Eine bevorzugte und insbesondere einfach und zweckmässig durchzuführende Verfahrensweise des erfindungsgemässen Verfahrens kann derart erfolgen, dass man die obengenannten Komponenten Phosphorsäure oder phosphorige Säure, Hydroxalkylamin der Formel (3) oder Glykol der Formel (5) und Amin der Formel (4) in den entsprechenden Mengen miteinander vermischt und sodann unter weiterem Rühren (Vermischen) auf etwa 180°C erwärmt. Diese Verfahrensschritte können auch kontinuierlich, beispielsweise in einem Strömungsrohr oder ähnlichen apparativen Vorrichtungen, vorgenommen werden. In der Regel beginnt bereits bei etwa 120 bis 150°C die Abscheidung von Wasser, das in den Einsatzprodukten enthalten ist. Mit fortschreitender Temperaturerhöhung und Reaktion erfolgt sodann die Abscheidung des bei der Kondensationsreaktion freiwerdenden Reaktionswassers, das in einer Destillationsvorlage aufgefangen werden kann.

Nach Beendigung der Reaktion kann die verwendete Phosphorsäure oder phosphorige Säure neutralisiert werden, vorzugsweise bis zur schwach alkalischen Reaktion, beispielsweise mit verdünnter Natronlauge, verdünnter Natriumcarbonatlösung oder mit Magnesiumoxid. Restliches Ausgangsamin der allgemeinen Formel (4), falls es in geringem Überschuss in das Reaktiongemisch eingesetzt wird, oder noch nicht vollständig umgesetztes, als Zwischenprodukt auftretendes $\beta$-hydroxyalkyliertes Amin kann danach durch Destillation, beispielsweise unter reduziertem Druck oder mit Wasserdampf, in an und für sich üblicher Weise entfernt werden. Aus der aus der Wasserdampfdestillation erhältlichen wässrigen Suspension bzw. Emulsion scheiden sich die hergestellten Verbindungen der allgemeinen Formel (1) beim Abkühlen ab, wobei sie, wie auch die durch Vakuumdestillation erhältlichen Verbindungen, abhängig von deren Konstitution und physikalischen Eigenschaften, entweder als gut kristallisierende und kristalline oder als ölige oder wachsartige Substanzen anfallen.

Erforderlichenfalls können diese erfindungsgemäss hergestellten Verbindungen nochmals gereinigt werden, beispielsweise durch fraktionierte Destillation unter reduziertem Druck oder durch Lösen in einer Säure, beispielsweise in verdünnter wässriger Salzsäure, und, gegebenenfalls nach einer Klärfiltration, durch anschliessende Fällung mit einer Lauge, beispielsweise mit Natronlauge, oder auch durch Umlösen der erhaltenen Verbindung aus einem Lösemittel, beispielsweise Äthanol oder Toluol, d.h. also durch Verfahren, wie sie im allgemeinen in der Technik üblich sind.

Die Ausbeuten des erfindungsgemässen Verfahrens zur Herstellung von Verbindungen der allgemeinen Formel (1) sind meist sehr gut und liegen in der Regel bei 90% der Theorie oder darüber.

Das erfindungsgemässe Verfahren hat gegenüber den oben erwähnten bekannten Verfahren neben der nach dem heutigen Stand der Erkenntnis physiologischen Unbedenklichkeit der Einsatzprodukte den erheblichen Vorzug, dass es bei wesentlich tieferen Reaktionstemperaturen durchgeführt werden kann, sehr hohe Ausbeuten liefert, insbesondere im Vergleich zu den bekannten Verfahren, und sich in Abwesenheit von brennbaren oder leicht entzündbaren organischen Lösemitteln durchführen lässt. Weiterhin bestehen bei der erfindungsgemässen Verwendung von Phosphorsäure oder phosphoriger Säure keine Korrosionsprobleme beim Arbeiten in Stahlgefässen. Die nach dem erfindungsgemässen Verfahren hergestellten Verbindungen der allgemeinen Formel (1) besitzen darüber hinaus nur eine sehr geringe Verfärbung und damit eine hohe Reinheit.

Bevorzugt nach dem erfindungsgemässen Verfahren ist die Herstellung der Amine der allgemeinen Formel (1), in welchen beide A gleich oder verschieden, bei den Verbindungen der Formel (6) bevorzugt gleich sind und jedes den Rest des Naphthalins oder Anthracens, insbesondere bevorzugt des Benzols, bedeutet, wobei bevorzugt die Naphthylreste, insbesondere aber die Phenylreste substituiert sein können, beispielsweise durch Substituenten, insbesondere 1 oder 2 Substituenten, aus der Gruppe Halogen, wie Fluor, Chlor und Brom, Alkyl, Alkoxy,

Hydroxy, Cyan, Nitro, Acylamino, Trifluormethyl, Carboxy, Carbalkoxy, Carbo-aryloxy, Carbamoyl, durch Alkyl, Phenyl, Benzyl und Phenäthyl mono- oder disubstituiertes Carbamoyl, Sulfo, Sulfamoyl, durch Alkyl, Phenyl, Benzyl und Phenäthyl mono- oder disubstituiertes Sulfamoyl und Aryloxy.

Von den oben erwähnten Substituenten, die Alkylreste enthalten, sind diejenigen Alkylreste enthaltenden Substituenten bevorzugt, deren Alkylreste aus 1 bis 6 C-Atomen, insbesondere aus 1 bis 4 C-Atomen bestehen. Arylreste in den oben erwähnten Substituenten sind bevorzugt der Phenylrest. Alkylreste in den obenerwähnten Substituenten sind insbesondere bevorzugt die Methyl- und Äthylgruppe; somit sind von diesen Substituenten, die einen Alkylrest besitzen, insbesondere die Methyl-, Äthyl-, Methoxy-, Äthoxy-, Carbomethoxy-, Carbäthoxy-, N-Methyl-carbamoyl-, N,N-Dimethylcarbamoyl- und N-Methyl-sulfamoyl-Gruppen zu erwähnen. Acylaminoreste sind insbesondere solche einer aliphatischen oder aromatischen Carbonsäure, wie beispielsweise der Benzoylaminorest oder der Acylaminorest einer Alkancarbonsäure von insgesamt 1 bis 6 C-Atomen, wie beispielsweise der Acetylamino- oder Propionylaminorest.

Bevorzugte Substituenten in den obengenannten Phenyl- und Naphthylresten, die für die beiden Formelglieder A stehen können, sind insbesondere die Methyl-, Äthyl-, Methoxy-, Äthoxy- und Nitrogruppe und das Chloratom. Insbesondere bevorzugt als Substituenten sind die Methyl-, Äthyl-, Methoxy- und Äthoxygruppe und das Chloratom zu nennen.

Alkylreste, die für die Formelreste $R^1$, $R^2$, $R^3$ und $R^4$ stehen können, sind bevorzugt Alkylgruppen von 1 bis 6 C-Atomen, insbesondere von 1 bis 4 C-Atomen, wobei diese Alkylreste substituiert sein können, auch durch eine Arylrest, wie beispielweise einen Phenylrest, und somit einen Aralkylrest darstellen. Alkylreste und substituierte Alkylreste sind insbesondere die Methyl-, Äthyl-, Butyl- und Benzylgruppe.

Arylreste, die für die Formelreste $R^1$, $R^2$, $R^3$ und $R^4$ stehen können, sind beispielsweise der Phenylrest, der beispielweise durch 1, 2 oder 3 Substituenten aus der Gruppe Alkyl von 1 bis 4 C-Atomen, Alkoxy von 1 bis 4 C-Atomen, Chlor, Brom, Trifluormethyl, Carbamoyl, Sulfamoyl, Carboxy und Sulfo substituiert sein kann.

Ein Cycloalkylrest, der für $R^1$, $R^2$, $R^3$ oder $R^4$ stehen kann, ist bevorzugt der Cyclohexylrest.

Als substituierter Alkylrest kann für die Formelreste $R^1$, insbesondere in der Ausgangsverbindung der Formel (4), und ebenso für den Formelrest $R^2$ und für den Formelrest $R^3$ auch die $\beta$-Hydroxyäthyl-Gruppe besonders erwähnt werden.

Bevorzugt ist die erfindungsgemässe Herstellung von Verbindungen der allgemeinen Formel (7)

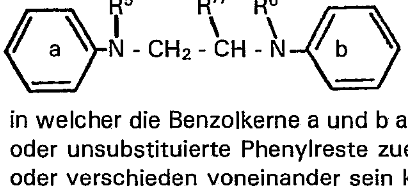

(7)

in welcher die Benzolkerne a und b als substituierte oder unsubstituierte Phenylreste zueinander gleich oder verschieden voneinander sein können, R'' für ein Wasserstoffatom, die Methyl- oder Äthylgruppe steht, $R^5$ und $R^6$ gleich oder verschieden voneinander sind und $R^5$ einen gegebenenfalls substituierten Alkyl-, Aralkyl-, Aryl- oder Cycloalkylrest, insbesondere einen der für $R^1$ oben erwähnten bevorzugten und besonders erwähnten Reste bedeutet und $R^6$ für ein Wasserstoffatom oder einen gegebenenfalls substituierten Alkyl-, Aralkyl-, Aryl- oder Cycloalkylrest, insbesondere für einen der oben für $R^1$ erwähnten bevorzugten oder besonders erwähnten Reste steht, durch Umsetzung von einer Verbindung der allgemeinen Formel (3c) mit einem Amin der allgemeinen Formel (4a)

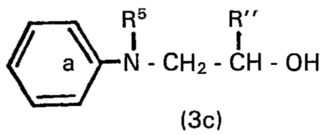

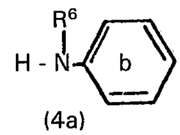

(3c) (4a)

in welchen die Benzolkerne a und b und R'', $R^5$ und $R^6$ die eben genannten Bedeutungen haben, in äquimolaren Mengen. Das Amin der Formel (4a) kann in geringem Überschuss eingesetzt werden. Insbesondere bevorzugt ist $R^5$ und $R^6$ jedes eine Methyl-, Äthyl-, Butyl-, Cyclohexyl- oder Benzylgruppe und $R^6$ bevorzugt ein Wasserstoffatom.

Sofern die vorstehend oder nachstehend beschriebenen Phenylreste a und b Substituenten enthalten, sind diese Substituenten bevorzugt Methyl, Äthyl, Methoxy, Äthoxy und Chlor.

Bevorzugt ist weiterhin die erfindungsgemässe Herstellung von Verbindungen der allgemeinen Formel (8)

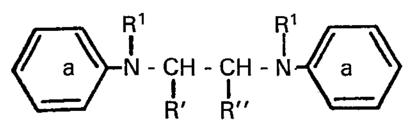

(8)

in welcher die Benzolkerne a als substituierte oder unsubstituierte Phenylreste zueinander gleich sind und $R^1$, jedes gleich zueinander, ein gegebenenfalls substituierter Alkyl-, Aralkyl-, Aryl- oder Cycloalkylrest, insbesondere ein Rest der für $R^1$ oben erwähnten bevorzugten und besonders erwähnten Bedeutung ist und R' und R'' die obigen Bedeutungen besitzen, durch Umsetzung von einem Mol der allgemeinen Formel (5) und etwa zwei Mol des Amins der allgemeinen Formel (4a)

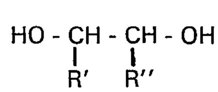

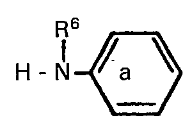

(5) (4a)

in welchen der Benzolkern a sowie $R^6$, R' und R'' die obengenannten Bedeutungen haben. Das Amin der Formel (4a) kann in geringem Überschuss eingesetzt werden.

Des weiteren ist bei der erfindungsgemässen Herstellung einer Triaryl-dialkylen-triamin-Verbindung der allgemeinen Formel (1), d.h. Verbindung der allgemeinen Formel (1), in welcher $R^1$ für einen Rest der Formel (2) steht, bevorzugt die Herstellung der

Verbindungen der allgmeinen Formel (9)

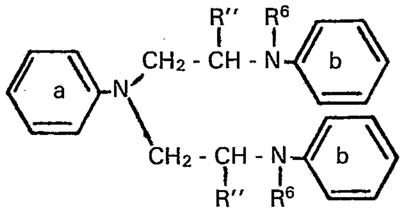

(9)

in welcher die Benzolkerne a und b sowie R'' und $R^6$ die obengenannten, insbesondere bevorzugten Bedeutungen besitzen, indem man ein Amin der obigen allgemeinen Formel (3c) und ein Amin der obengenannten allgemeinen Formel (4a), in welchen die Benzolkerne a und b sowie R'' die obengenannten Bedeutungen haben, der Rest $R^6$ die obengenannte Bedeutung ausser Wasserstoff besitzt und $R^5$ für eine entsprechende $\beta$-Hydroxyalkyl-Gruppe steht, miteinander im molaren Verhältnis von 1 : 2, gegebenenfalls mit einem geringen Überschuss an der Verbindung der Formel (4a), umsetzt. Hierin haben die Phenylreste a und b gleiche oder verschiedene Bedeutung und enthalten, falls sie substituiert sind, bevorzugt als Substituenten ein Chloratom, eine Methyl-, Äthyl-, Methoxy- oder Äthoxygruppe; R'' ist jedoch bevorzugt ein Wasserstoffatom, und $R^6$ ist bevorzugt eine Alkylgruppe von 1 - 4 C-Atomen oder die Benzylgruppe.

Die einfache und glatte Herstellung dieser Triaryldiäthylen-triamin-Verbindung nach dem erfindungsgemässen Verfahren, ausgehend von den N,N-Di-($\beta$--hydroxyalkyl)-arylaminen unter der kondensierenden Wirkung von Phosphorsäure oder phosphoriger Säure überrascht, da es aus der oben erwähnten Veröffentlichung J. Am. Chem. Soc. *42*, 1725 (1920) bekannt ist, dass N,N-Di-($\beta$-hydroxyäthyl)-anilin unter der kondensierenden Wirkung von Phosphorpentoxid einen intramolekularen Ringschluss zum N-Phenylmorpholin unter Wasserabspaltung eingeht.

Weiterhin bevorzugt ist die erfindungsgemässe Herstellung von Piperazin-Verbindungen entsprechend der allgemeinen Formel (1), die die allgemeinen Formeln (6c) und (6d)

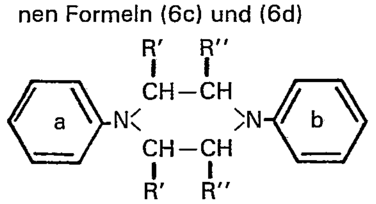

(6c)

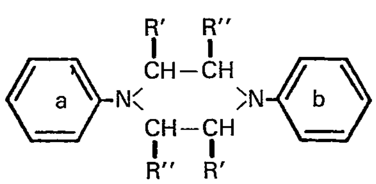

(6d)

besitzen, in welchen R' und R'' sowie die Benzolkerne a und b wie oben definiert sind.

Gemäss dem erfindungsgemässen Verfahren geht man im insbesonders bevorzugten Falle, dass a und b zueinander gleiche Bedeutungen besitzen, von äquimolaren Mengen der Ausgangsverbindungen entsprechend der obigen Formeln (5) und (4a) aus, wobei das Amin der Formeln (4a) in einem geringen Überschuss eingesetzt werden kann.

Gemäss dem erfindungsgemässen Verfahren geht man im ebenso insbesondere bevorzugten Falle, im welchem R' und R'' jedes für ein Wasserstoffatom steht und a und b gleiche oder verschiedene Bedeutungen besitzen, von äquimolaren Mengen der Ausgangsverbindungen der Formeln (3d) und (3e)

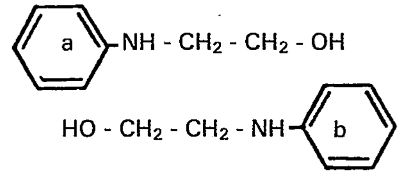

(3d)

(3e)

mit a und b der obengenannten Bedeutung aus; haben a und b die gleiche Bedeutung, so setzt man irgendeine Menge dieses Amins in das erfindungsgemässe Verfahren ein und kommt somit zu den symmetrischen N,N'-disubstituierten Piperazinverbindungen. Zu Verbindungen der allgemeinen Formeln (6) kann man auch gelangen, wenn man eine Verbindung der allgemeinen Formel (3) und insbesondere (3c), in welchen A bzw. der Phenylrest a die obengenannte Bedeutung hat und $R^3$ und $R^5$ jedes für eine $\beta$-Hydroxyalkyl-, bevorzugt die $\beta$-Hydroxyäthyl-Gruppe stehen, mit der äquimolaren Menge des aromatischen Amins der allgemeinen Formel (4) und insbesondere (4a), in welchen A bzw. der Phenylrest b die obengenannten Bedeutungen haben und $R^4$ und $R^6$ jedes für ein Wasserstoffatom stehen, in äquimolaren Mengen in erfindungsgemässer Weise umsetzt.

Die Verbindungen der allgemeinen Formel (1) dienen als wertvole Zwischenprodukte je nach ihrer Konstitution zur Herstellung von Pflanzenschutzmitteln, Textilhilfsmitteln, von Farbstofffen und Farbstoffvorprodukten. So eignen sie sich, wie allgemein von anderen Arylaminen bekannt, insbesondere wenn sie in p-Stellung zur Aminogruppe nicht substituiert sind, als Ausgangsprodukte zur Herstellung von Azofarbstoffen, von Triarylmethanfarbstoffen, von Methin-, Azomethin- und Anthrachinonfarbstoffen.

Die nachstehenden Beispiele dienen zur Erläuterung des Erfindungsgegenstandes. Die Teile sind Gewichtsteile, die Prozentangaben beziehen sich auf Gewichtsprozente. Gewichtsteile verhalten sich zu Volumenteilen wie Kilogramm zu Liter.

*Beispiel 1*

151 Teile N-Methyl-N-($\beta$-hydroxyäthyl)-anilin, 128 Teile N-Methylanilin und 38 Teile 85%ige Phosphorsäure werden miteinander vermischt und unter gutem Rühren innerhalb etwa 1 Stunde auf 180°C erwärmt. Bei etwa 150°C beginnt die Wasserabscheidung, das in einer Destillationsvorlage aufgefangen werden kann; mit dem Wasser geht etwas N-Methylanilin über. Man hält das Reaktionsgemisch insgesamt etwa 8 Stunden bei der Reaktionstemperatur von etwa 180°C; während dieser Zeit scheiden sich insgesamt 23 Teile Wasser und etwa 6 bis 7 Volumenteile N-Methylanilin ab. Nach dieser Reaktionszeit ist im Dünnschichtchromatogramm kein N-Methyl-N-($\beta$-hydroxyäthyl)-anilin mehr nachweisbar. Man kühlt das Reaktionsgemisch auf 80°C ab, gibt

verdünnte Natronlauge bis zur schwach alkalischen Reaktion hinzu und destilliert mit Wasserdampf, bis kein N-Methylanilin mehr übergeht.

Den Rückstand der Wasserdampfdestillation giesst man in etwa 2000 Teile kaltes Wasser, wobei die anfänglich ölige Suspension kristallin erstarrt. Man saugt ab und trocknet bei etwa 30 - 35°C. Man erhält 218 Teile N,N'-Dimethyl-N,N'-diphenyl-äthylendiamin der Formel

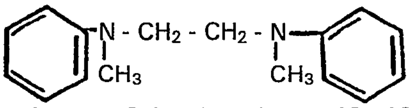

mit einem Schmelzpunkt von 42 - 46°C. Eine Probe wird in Methanol umgelöst; sie schmilzt bei 48 - 50°C. Die Ausbeute beträgt etwa 90% d.Th.

*Beispiel 2*

165 Teile N-Äthyl-N-(β-hydroxyäthyl)-anilin, 128 Teile N-Methylanilin und 19 Teile 85%ige Phosphorsäure werden miteinander vermischt und unter gutem Rühren innerhalb 1 Stunde auf etwa 180°C erhitzt. Die Reaktion wird dann in analoger Weise, wie im Beispiel 1 beschrieben, weitergeführt. In der Destillationsvorlage scheiden sich etwa 20,5 Teile Wasser und 6 - 7 Volumenteile N-Methylanilin ab. Nach Beendigung der Reaktion kühlt man das Reaktionsgemisch auf etwa 80°C ab, neutralisiert bis zur schwach alkalischen Reaktion mit verdünnter Natronlauge und entfernt überschüssiges Methylanilin mit Hilfe der Wasserdampfdestillation. Sodann rührt man 2000 Teile kaltes Wasser hinzu; es scheidet sich ein Öl ab, das abgetrennt und durch Vakuumdestillation gereinigt wird. Die Hauptfraktion destilliert bei einem Druck von 2,4 mbar bei einer Siedetemperatur von 180°C über. Das im Dünnschichtchromatogramm sich einheitlich zeigende Produkt erstarrt nach einiger Zeit zu einer wachsartigen, kristallinen Masse, die bei etwa 35°C schmilzt. Die Ausbeute an dem N-Methyl-N'-äthyl-N,N'-diphenyl-äthylendiamin der Formel

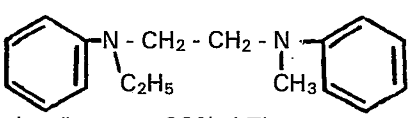

beträgt etwa 90% d.Th.

*Beispiel 3*

165 Teile N-Äthyl-N-(β-hydroxyäthyl)-anilin, 127 Teile N-Äthylanilin und 12 Teile 85%ige Phosphorsäure werden nach Vermischen etwa 15 Stunden bei 180°C erhitzt. In der Destillationsvorlage scheiden sich etwa 20 Teile Wasser und etwa 2 bis 3 Teile einer Mischung aus den beiden als Ausgangsverbindungen eingesetzten Anilinverbindungen ab. Nach Beendigung der Reaktion lässt man das Reaktionsgemisch auf etwa 80°C abkühlen, stellt mit Natronlauge sschwach alkalisch und entfernt den geringen Überschuss an N-Äthylanilin mittels einer Wasserdampfdestillation. Sodann werden 2000 Teile kaltes Wasser eingerührt, wobei sich das gebildete N,N'-Diäthyl-N,N'-diphenyläthylendiamin der Formel

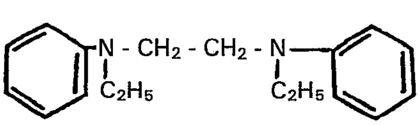

in einer farblosen Kristallmasse abscheidet. Diese wird abgesaugt, mit kaltem Wasser neutral gewaschen und bei etwa 40°C getrocknet. Man erhält 244 Teile helle Kristalle mit einem Schmelzpunkt von 65 - 69°C. Diese Kristallmasse kann aus Äthanol umgelöst werden, wobei man die Verbindung mit einem Schmelzpunkt von 75°C erhält. Die Ausbeute beträgt etwa 90% d.Th.

*Beispiel 4*

195 Teile N,N-Di-(β-hydroxyäthyl)-4-methyl-anilin, 320 Teile N-Methylanilin und 38 Teile 85%ige Phosphorsäure werden nach Mischung etwa 15 Stunden lang bei einer Temperatur von 180 - 200°C erhitzt; im Verlaufe der Reaktion scheiden sich etwa 42 Teile Wasser und 15 Volumenteile N-Methylanilin ab. Aus der dünnschichtchromatographischen Prüfung ergibt sich, dass das Reaktionsgemisch die oben erwähnte Hydroxyäthyl-Verbindung nicht mehr enthält. Es wird auf 80°C abgekühlt, mit Natronlauge schwach alkalisch gestellt und einer Wasserdampfdestillation zur Entfernung des N-Methylanilins unterworfen. Sodann werden etwa 3000 Teile kaltes Wasser eingerührt, wobei sich nach einiger Zeit eine halbfeste Masse abscheidet. Sie wird vom Wasser abgetrennt, neutral gewaschen und getrocknet. Es werden 339 Teile eines Rohproduktes erhalten, das aus Äthanol umgelöst bei 113 bis 115°C schmilzt.

Die Analyse ergibt folgende Werte:

| | | | | |
|---|---|---|---|---|
| ber.: | C 80,43% | gef.: | C 80,4%, | 80,2% |
| | H 8,31% | | H 8,3%, | 8,4% |
| | N 11,26% | | N 11,3%, | 11,1% |

Die Substanz enthält keinen Sauerstoff.

Die Analysenwerte entsprechen der hergestellten Verbindung der Formel

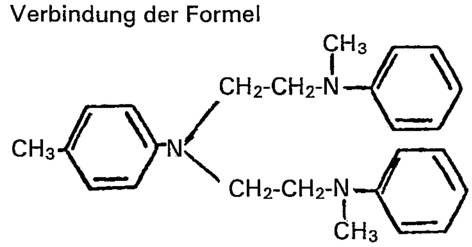

mit der chemischen Bezeichnung N,N''-Dimethyl-N,N''-diphenyl-N'-(4-methylphenyl)-diäthylen-triamin. Die Ausbeute beträgt etwa 90% d.Th.

*Beispiel 5*

274 Teile N-(β-Hydroxyäthyl)-anilin und 38 Teile Phosphorsäure werden auf 180°C erhitzt. Diese Reaktionstemperatur wird noch etwa 6 Stunden beibehalten. Es scheiden sich in der Destillationsvorlage etwa 41 Teile Wasser ab; mit ihm geht eine sehr geringe Menge an N-(β-Hydroxyäthyl)-anilin über. Das Reaktionsgemisch kann beim Erkalten erstarren. Aus diesem Grunde wird es noch in der Wärme in ein Gemisch aus 2000 Teilen Wasser und 80 Volumenteilen einer 33%igen Natronlauge eingerührt. Es scheiden sich helle Kristalle ab, die abgesaugt und getrocknet werden. Man erhält 230 Teile N,N'-Diphenylpiperazin der Formel

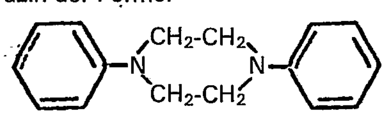

mit einem Schmelzpunkt von 163°C.

Diese Verbindung kann auch entsprechend der Verfahrensweise, wie im nachfolgenden Beispiel 6 beschrieben, hergestellt werden.

*Beispiel 6*

181 Teile N,N-Di-(β-hydroxyäthyl)-anilin, 99 Teile Anilin und 38 Teile einer 85%igen Phosphorsäure werden vermischt und auf 180°C erhitzt. Die Reaktion wird etwa 6 Stunden bei dieser Temperatur weitergeführt, wobei sich in einer Destillationsvorlage 41 Teile Wasser und 6 Volumenteile Anilin abscheiden. Das Reaktionsprodukt erstarrt beim Abkühlen; die Reaktionsmischung wird deshalb noch heiss auf ein Gemisch aus 1000 Teilen Wasser und 80 Volumenteilen einer 33%igen Natronlauge eingerührt. Es scheidet sich eine helle Kristallmasse aus, die abgesaugt, gewaschen und getrocknet wird. Die Ausbeute beträgt 225 Teile. Dieses so hergestellte N,N'-Diphenylpiperazin wird aus Toluol oder Xylol umgelöst. Man erhält farblose Kristalle mit einem Schmelzpunkt von 163°C, die mit der gemäss Beispiel 5 erhaltenen Verbindung keine Schmelzpunktdepression zeigen. Ausbeute der reinen Substanz: 85% d.Th.

*Beispiel 7*

181 Teile N,N-Di-(β-hydroxyäthyl)-anilin, 113 Teile 3-Methylanilin und 38 Teile einer 85%igen Phosphorsäure werden zusammen etwa 6 Stunden lang bei 180°C erhitzt. Es scheiden sich 41 Teile Wasser und 6 Volumenteile 3-Methylanilin ab. Das Reaktionsgemisch wird noch heiss in ein Gemisch aus 1000 Teilen Wasser und 80 Volumenteilen einer 33%igen Natronlauge eingerührt. Es scheidet sich eine helle Kristallmasse aus, die abgesaugt und bei 60°C getrocknet wird. Es werden 238 Teile erhalten. Dieses Rohprodukt ergibt beim Umlösen aus Aceton farblose Kristalle des N--Phenyl-N'-(3-methylphenyl)-piperazin mit einem Schmelzpunkt von 129 - 130°C. Ausbeute: 90% d.Th.

*Beispiel 8*

*195 Teile 3-Methyl-N,N-di-(β-*hydroxyäthyl)-anilin, 99 Teile Anilin und 38 Teile einer 85%igen Phosphorsäure werden während 6 Stunden bei 180°C erhitzt. Es scheiden sich 41 Teile Wasser und 6 Volumenteile Anilin ab. Da das Reaktionsprodukt beim Abkühlen erstarrt, wird die Reaktionsmischung noch heiss auf ein Gemisch aus 1000 Teilen Wasser und 80 Volumenteilen einer 33%igen Natronlauge gerührt. Es scheidet sich eine helle Kristallmasse ab, die abgesaugt, gewaschen und bei 60°C getrocknet wird. Man erhält 240 Teile N-Phenyl-N'-(3-methylphenyl)-piperazin. Es ist mit der gemäss Beispiel 7 hergestellten Piperazinverbindung identisch. Es kann aus Aceton umgelöst werden und zeigt einen Schmelzpunkt von 129°C. Mit der nach Beispiel 7 hergestellten Verbindung zeigt sie keine Schmelzpunktsdepression. Ausbeute: ca. 90% d.Th.

*Beispiel 9*

195 Teile 3-Methyl-N,N-di-(β-hydroxyäthyl)-anilin, 113 Teile 3-Methylanilin und 38 Teile 85%iger Phosphorsäure werden vermischt und etwa 6 Stunden auf 180 - 190°C erhitzt. Es scheiden sich 41 Teile Wasser und 6 Volumenteile 3-Methylanilin ab. Die hoch heisse Reaktionsmischung, die beim Abkühlen erstarren kann, wird in ein Gemisch aus 1000 Teilen Wasser und 80 Volumenteilen einer 33%igen Natronlauge eingerührt. Es scheidet sich eine helle Kristallmasse ab, die abgesaugt, gewaschen und getrocknet wird. Man erhält 240 Teile eines Produktes, das aus Aceton umgelöst farblose Kristalle der Verbindung der Formel

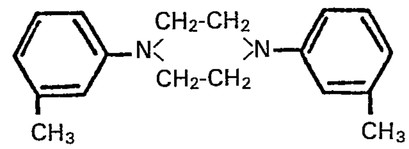

mit einem Schmelzpunkt von 125 - 126°C ergibt. Ausbeute der reinen Substanz: 90% d.Th.

*Beispiele 10 bis 37*

Verfährt man in erfindungsgemässer Weise, beispielsweise analog einer der in den Ausführungsbeispielen beschriebenen Verfahrensweisen, so lassen sich bei Wahl der in den nachfolgenden Tabellenbeispielen erwähnten Ausgangsverbindungen die entsprechenden Äthylendiamine der allgemeinen Formel (1) ebenfalls mit sehr guter Ausbeute und in guter Reinheit herstellen. In manchen Fällen fallen die Produkte ölig oder wachsartig an.

Es werden zuerst tabellarisch die Endprodukte der allgemeinen Formel (1) wiedergegeben und nachfolgend in einer gesonderten Tabelle die hierfür eingesetzten Ausgangsverbindungen.

| Bsp. | Verbindung der Formel (1) |
|---|---|
| 10 | 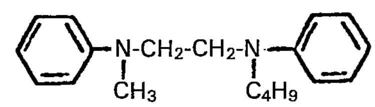 |
| 11 | 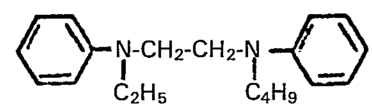 |
| 12 | 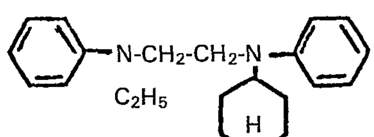 |
| 13 | 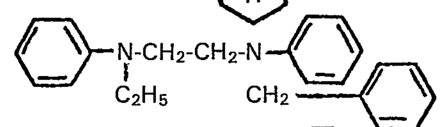 |
| 14 | 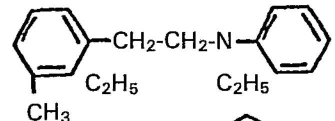 |
| 15 | 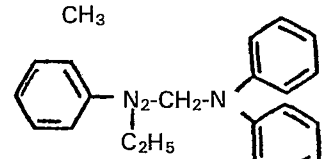 |
| 16 | 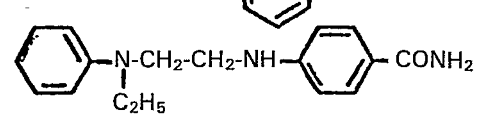 |

| Bsp. | Verbindung der Formel (1) |
|---|---|
| 17 | 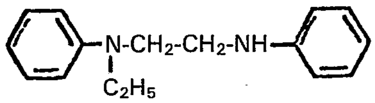 |
| 18 | 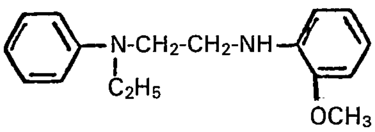 |
| 19 | 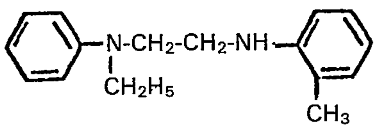 |
| 20 | 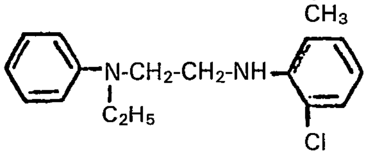 |
| 21 | 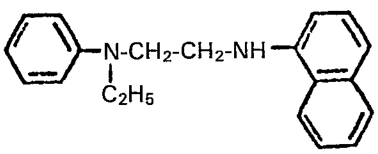 |
| 22 | 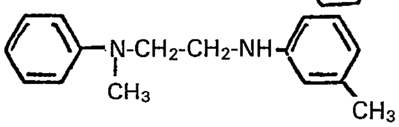 |
| 23 | 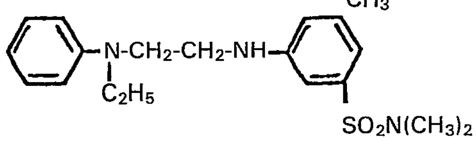 |
| 24 | 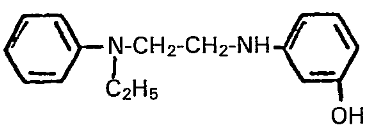 |
| 25 | 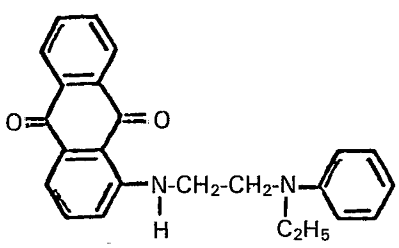 |
| 26 | 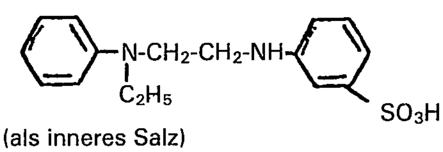 (als inneres Salz) |
| 27 | 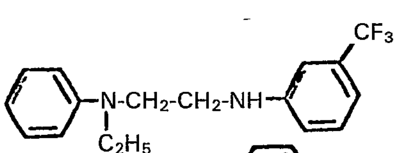 |
| 28 | 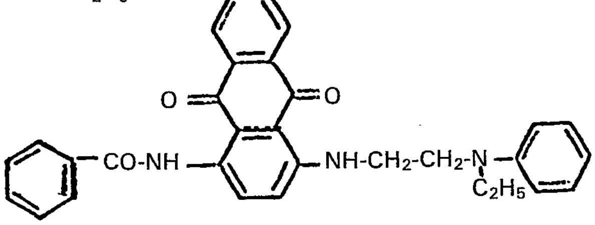 |

| Bsp. | Verbindung der Formel (1) | Smp. (°C) |
|---|---|---|
| 29 | 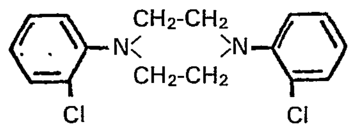 | 180 |
| 30 | 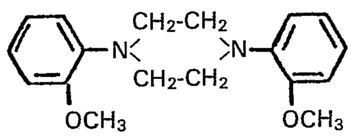 | 139 |
| 31 | 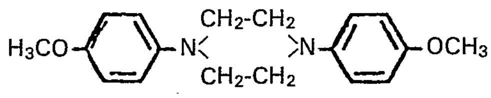 | 233 |
| 32 | 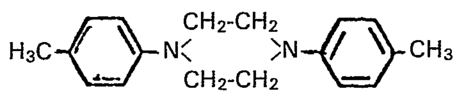 | 189 |
| 33 | 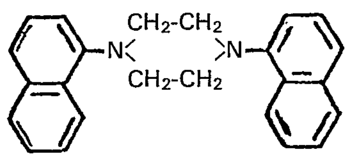 | 180 |
| 34 | 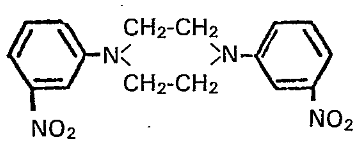 | 220 |
| 35 | 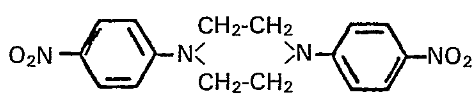 | 248 |
| 36 | 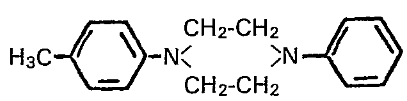 | |
| 37 | 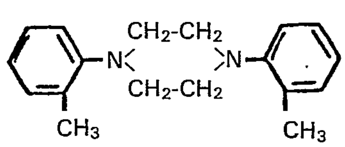 | 174 |

| Bsp. | hergestellt aus äquimolaren Mengen der Verbindung (3) | und der Verbindung (4) | Reaktions-temp. (°C) | Menge an $H_3PO_4$ (1: . . . Mol) |
|---|---|---|---|---|
| 10 | N-Methyl-N-(β-hydroxy-äthyl)-anilin | N-n-Butyl-anilin | 190 | 0,15 |
| 11 | N-Äthyl-N-(β-hydroxy-äthyl)-anilin | dito | 185 | 0,15 |
| 12 | dito | N-Cyclohexyl-anilin | 190 | 0,15 |
| 13 | dito | N-Benzyl-anilin | 180 | 0,18 |
| 14 | 3-Methyl-N-äthyl-N-(β-hydroxy-äthyl)-anilin | N-Äthyl-anilin | 180 | 0,14 |
| 15 | N-Äthyl-N-(β-hydroxy-äthyl)-anilin | Diphenylamin | 180 | 0,15 |
| 16 | dito | 4-Carbamoyl-anilin | 180 | 0,15 |
| 17 | dito | Anilin | 180 | 0,18 |
| 18 | dito | 2-Methoxyanilin | 185 - 190 | 0,20 |
| 19 | dito | 2-Methylanilin | 180 | 0,25 |
| 20 | dito | 2-Chlor-anilin | 200 | 0,15 |
| 21 | dito | 1-Naphthylamin | 190 | 0,15 |
| 22 | N-Methyl-N-(β-hydroxy-äthyl)-anilin | 3-Methylanilin | 180 | 0,12 |
| 23 | N-Äthyl-N-(β-hydroxy-äthyl)-anilin | 3-(N',N'-Dimethyl-sulfamoyl)-anilin | 180 - 190 | 0,15 |
| 24 | dito | 3-Aminophenol | 180 | 0,20 |
| 25 | dito | 1-Amino-anthrachinon | 180 | 0,22 |
| 26 | dito | 3-Sulfo-anilin | 200 | 0,15 |
| 27 | dito | 3-Trifluormethyl-anilin | 190 | 0,15 |
| 28 | N-Äthyl-N-(β-hydroxy-äthyl)-anilin | 1-Amino-4-benzoyl-amino-anthrachinon | 190 | 0,15 |
| 29 | 2-Chlor-N,N-di-(β-hydroxy-äthyl)-anilin | 2-Chlor-anilin | 180 | 0,20 |
| 30 | 2-Methoxy-N-(β-hydroxy-äthyl)-anilin | wie (3) | 180 | 0,20 |
| 31 | 4-Methoxy-N-(β-hydroxy-äthyl)-anilin | wie (3) | 180 | 0,25 |
| 32 | 4-Methyl-N-(β-hydroxy-äthyl)-anilin | wie (3) | 190 | 0,12 |
| 33 | 1-N,N-Di-(β-hydroxy-äthyl)-naphthylamin | 1-Naphthylamin | 180 - 190 | 0,15 |
| 34 | 3-Nitro-N-(β-hydroxy-äthyl)-anilin | wie (3) | 200 | 0,15 |
| 35 | 4-Nitro-N-(β-hydroxy-äthyl)-anilin | wie (3) | 190 | 0,20 |
| 36 | 4-Methyl-N,N-di-(β-hydroxy-äthyl)-anilin | Anilin | 180 | 0,18 |
| 37 | 2-Methyl-N-(β-hydroxy-äthyl)-anilin | wie (3) | 180 | 0,15 |

*Beispiel 38*

Man verfährt wie im Bsp. 4 beschrieben, setzt jedoch 195 Teile N,N-Di-(β-hydroxyäthyl)-3-methyl-anilin, 320 Teile N-Methylanilin und 38 Teile 85%ige Phosphorsäure ein. Man erhält das N,N''-Dimethyl-N,N''-diphenyl-N'-(3-methylphenyl)-diäthylen-triamin der Formel

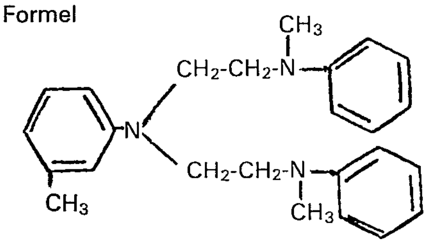

in farblosen Kristallen mit einem Schmelzpunkt von 127 - 129°C.

Die Analyse ergab folgende Werte:

| | | | | |
|---|---|---|---|---|
| ber.: | C 80,43% | gef.: | C 80,5%, | 80,5% |
| | H 8,31% | | H 7,8%, | 8,0% |
| | N 11,26% | | N 11,4%, | 11,3% |

die Substanz enthält keinen Sauerstoff.

*Beispiel 39*

364 Teile N,N-Di-(β-hydroxyäthyl)-anilin, 470 Teile N-Methylanilin und 153 Teile 85%ige Phosphorsäure werden miteinander gemischt und unter Rühren auf etwa 185°C erwärmt. Im Verlauf von etwa 10 Stunden werden 109 Teile Wasser und etwa 10 Teile N-Methylanilin abdestilliert. Wenn kein Wasser mehr übergeht, ist im Dünnschichtchromatogramm auch kein Dihydroxyäthylanilin als Ausgangsprodukt mehr nachweisbar. Man neutralisiert mit verdünnter Natronlauge bis zur schwach alkalischen Reaktion und destilliert das überschüssige N-Methylanilin mit Wasserdampf ab. Sodann trennt man die organische Phase von der Wasserphase ab und wäscht sie zweimal mit 2000 Teilen Wasser aus; man erhält etwa 655 Teile eines öligen/wachsartigen Rohproduktes, das, mit etwa 2000 Teilen Äthanol ausgerührt, farblose Kristalle mit einem Schmelzpunkt von 164 - 168°C ergibt. Eine nochmalige Umkristallisation aus heissem Äthanol oder Toluol ergibt ein Produkt mit einem Schmelzpunkt von 167 - 170°C.

Analyse:

| | | | | |
|---|---|---|---|---|
| ber.: | C 80,22% | gef.: | C 80,4%, | 80,6% |
| | H 8,08% | | H 7,8%, | 7,7% |
| | N 11,70% | | N 11,9%, | 11,6% |

die Substanz enthält keinen Sauerstoff.

Die Analysenwerte des Produktes entsprechen dem N,N''-Dimethyl-N,N',N''-triphenyl-diäthylen-triamin der Formel

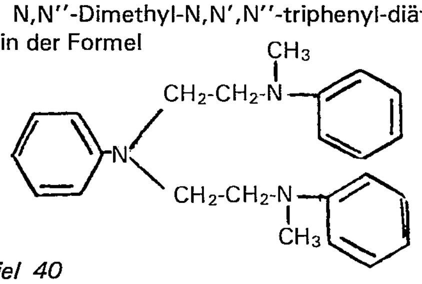

*Beispiel 40*

Man verfährt gemäss der Arbeitsweise des Beispieles 39, ersetzt jedoch die als Ausgangsverbindungen dienenden Anilinverbindungen durch äquivalente Mengen an N,N-Di-(β-hydroxyäthyl)-4-methyl-anilin und N-Äthylanilin; man erhält in entsprechender Weise die Verbindung der Formel

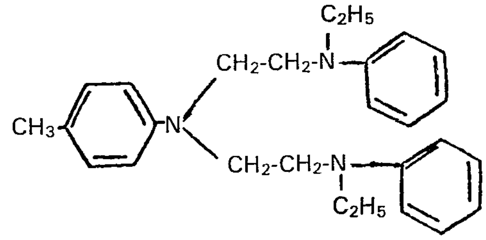

in etwa gleich guter Ausbeute und Reinheit.

*Beispiel 41*

195 Teile Anilin, 62 Teile Äthylenglykol ($HO-CH_2-CH_2-OH$) und 25 Teile einer wässrigen 85%igen Phosphorsäure werden gemischt und unter gutem Rühren innerhalb von einer Stunde auf 180°C erhitzt. Bei etwa 150°C beginnt sich Wasser abzuscheiden; mit ihm geht etwas Anilin über. Nachdem man die Reaktionsmischung etwa 8 Stunden bei 180 bis 190°C gerührt hat, haben sich in der Destillationsvorlage 39 Teile Wasser und 7 Teile Anilin abgeschieden. Nach dieser Reaktionszeit ist im Reaktionsgemisch dünnschichtchromatographisch nur noch eine Spur Anilin und N-β-Hydroxyäthylanilin neben dem Hauptprodukt, dem 1,2-Diphenylaminoäthan, und neben einer geringen Menge N,N'-Diphenylpiperazin nachweisbar. Man kühlt das Reaktionsgemisch auf 80 bis 90°C ab, gibt verdünnte Natronlauge bis zur schwach alkalischen Reaktion hinzu und destilliert mit Wasserdampf, bis kein Anilin und N-β-Hydroxyäthylanilin mehr übergehen. Danach gib man den Reaktionsansatz unter Rühren auf etwa 1000 Teile kaltes Wasser und isoliert das abgeschiedene duktile, wachsartige Reaktionsprodukt (etwa 210 Teile). Man verrührt es mit 500 Teilen Athanol und isoliert etwa 19 Teile farblose Kristalle, die bei 162 bis 163°C schmelzen und fast reines Diphenylpiperazin als bei der Kondensationsreaktion gebildetes Nebenprodukt darstellen. Der nach dem Abdampfen des Äthanols erhaltene Rückstand erstarrt zu einer fast farblosen Kristallmasse, die bei 60 bis 62°C schmilzt und neben einer Spur Diphenylpiperazin aus 1,2-Diphenylaminoäthan der Formel

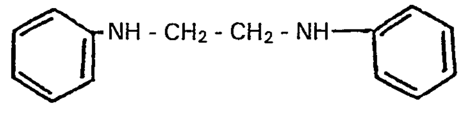

besteht. Ausbeute: 191 Teile.

Eine Probe wird aus etwa 80%igem Äthanol umgelöst; sie bildet farblose Kristalle, die einen Schmelzpunkt von 64 bis 65°C zeigen (Beilstein, Handbuch der Organischen Chemie, *12*, 543, gibt einen Festpunkt von 64°C und von 65°C an).

*Beispiel 42*

Eine Mischung aus 540 Teilen o-Anisidin, 124 Teilen Äthylenglykol und 50 Teilen einer 85%igen wässrigen Phosphorsäure wird unter gutem Rühren auf 180 bis 190°C erwärmt. Nach etwa 8 Stunden haben sich in der Destillationsvorlage etwa 80 Teile Wasser und 10 Teile o-Anisidin abgeschieden. Die beim Abkühlen erstarrende Reaktionsmasse wird nach der Zugabe von 20 Teilen Magnesiumoxid noch etwa 30 Minuten bei 120°C gerührt; danach wird das gebildete Magnesiumphosphat bei 100 bis 120°C über eine erwärmte Nutsche abgesaugt. Man

destilliert dann mit Wasserdampf das nicht umgesetzte o-Anisidin und gebildetes und nicht weiter umgesetztes N-β-Hydroxyäthyl-o-anisidin ab, trennt den beim Erkalten erstarrten Rückstand von der wässrigen Phase ab, wäscht mit etwa 100 Teilen Wasser nach und digeriert sodann mit etwa 500 Teilen Äthanol, wobei das anfänglich duktile Produkt zu einem farblosen Kristallbrei zerfällt. Man saugt ihn ab und erhält nach dem Trocknen 496 Teile fast farbloser Kristalle, die bei 113 bis 114°C schmelzen. Dünnschichtchromatographisch sind nur Spuren von 3 Verunreinigungen nachzuweisen.

Eine Probe der Kristalle wird aus Äthanol umgelöst; sie bildet farblose Kristalle von einem Smp. von 114°C, die dünnschichtchromatographisch eine einheitliche Verbindung darstellen. Die Analyse dieser neuen Verbindung zeigte folgende Werte:

| | | | |
|---|---|---|---|
| ber. C | 70,58% | gef.: C | 70,4% |
| H | 7,35% | H | 7,6% |
| N | 10,29% | N | 10,3% |
| O | 11,76% | O | 11,4% |
| $OCH_3$ | 22,79% | $OCH_3$ | 22,5% |

Die Analysenwerte entsprechen der erfindungsgemäss hergestellten neuen Verbindung der Formel

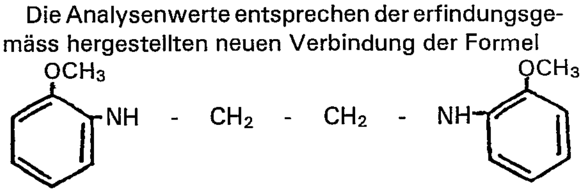

mit der chemischen Bezeichnung N,N'-Bis-(2--methoxyphenyl)-äthylendiamin.

*Beispiel 43*

Eine Mischung aus 600 Teilen o-Phenetidin, 124 Teilen Äthylenglykol und 50 Teilen einer wässrigen 85%igen Phosphorsäure wird unter gutem Rühren auf 180 bis 190°C erhitzt. Nach etwa 8 Stunden haben sich in der Destillationsvorlage etwa 80 Teile Wasser und 6 Teile o-Phenetidin abgeschieden. Da die Reaktionsmischung beim Abkühlen erstarrt, wird sie noch bei 120°C mit 20 Teilen Magnesiumoxid versetzt und in der Wärme nach etwa 30 Minuten über einer erwärmten Nutsche von dem gebildeten Magnesiumphosphat abfiltriert. Überschüssiges o-Phenetidin und wenig N-β-Hydroxyäthyl-o-phenetidin werden mit Wasserdampf abgetrieben; den festgewordenen Destillationsrückstand trennt man von der wässrigen Phase ab und wäscht ihn mit 200 bis 300 Teilen Wasser nach. Beim Digerieren mit 500 Teilen Äthanol zerfällt das duktile Reaktionsgemisch zu einer fast farblosen Kristallmasse, die abgesaugt und getrocknet wird. Man erhält etwa 542 Teile fast farblose Kristalle, die bei 111 bis 112°C schmelzen und dünnschichtchromatographisch nur Spuren von drei Nebenprodukten zeigen. Eine Probe wird aus Äthanol umgelöst und liefert farblose Kristalle, die dünnschichtchromatographisch ein einheitliches Produkt darstellen.

Diese neue Verbindung N,N'-Bis-(2-äthoxyphenyl)-äthylendiamin besitzt einen Smp. von 112 bis 113°C und zeigte die folgende Elementaranalyse:

| | | | |
|---|---|---|---|
| ber.: C | 72,00% | gef.: C | 71,8% |
| H | 8,00% | H | 8,1% |
| N | 9,33% | N | 9,3% |
| O | 10,67% | O | 10,2% |
| $OC_2H_5$ | 30,00% | $OC_2H_5$ | 29,6% |

*Beispiel 44*

Eine Mischung aus 530 Teilen p-Xylidin, 124 Teilen Äthylenglykol und 25 Teilen einer 85%igen wässrigen Phosphorsäure wird 10 Stunden lang bei 180 bis 190°C gerührt. In der Destillationsvorlage werden dabei 79 Teile Wasser und etwa 5 Teile p-Xylidin aufgefangen. Anschliessend gibt man bei einer Temperatur von 100 bis 120°C 10 Teile Magnesiumoxid hinzu und saugt nach 30 Minuten über eine erwärmte Nutsche vom Magnesiumphosphat ab. Die Reaktionsmasse wird sodann unter reduziertem Druck destilliert, wobei zunächst nicht umgesetztes p-Xylidin und etwas N-β-Hydroxyäthyl-p-xylidin übergehen; bei einer Sumpftemperatur von 230 bis 237°C mit einer Kopftemperatur von 220 bis 223°C und einem Druck von 2,3 bis 3,3 mbar erhält man sodann das gewünschte Reaktionsprodukt, das in der Vorlage beim Abkühlen kristallin erstarrt und dünnschichtchromatographisch einheitlich ist.

Die neue Verbindung N,N'-Bis-(2,5-dimethylphenyl)-äthylendiamin wird in einer Ausbeute von 540 Teilen erhalten. Fast farblose kristalline Masse, Smp.: 102 bis 103°C.

Analyse:

| | | | |
|---|---|---|---|
| ber.: C | 80,59% | gef.: C | 80,5% |
| H | 8,95% | H | 8,9% |
| N | 10,44% | N | 10,3% |
| O | 0,00% | O | 0,0% |

*Beispiel 45*

Eine Mischung aus 267 Teilen N-Äthylanilin, 62 Teilen Äthylenglykol und 38 Teilen einer wässrigen 85%igen Phosphorsäure wird unter Rühren auf 180 bis 190°C geheizt und bei dieser Temperatur 8 Stunden lang gerührt. Hierbei gehen 42 Teile Wasser und etwa 10 Teile N-Äthylanilin über. Sodann kühlt man auf 80°C ab, stellt das Reaktionsprodukt mit verdünnter wässriger Natronlauge schwach alkalisch und unterwirft es sodann einer Wasserdampfdestillation so lange, bis kein Öl [Äthylanilin und etwas N--(β-Hydroxyäthyl)-äthylanilin] mehr übergeht. Den Rückstand der Wasserdampfdestillation giesst man auf 2000 Teile kaltes Wasser; hierbei erstarrt das Reaktionsprodukt zu einem Kristallgranulat. Man saugt ab und wäscht mit Wasser neutral und trocknet das Produkt an der Luft bis zur Gewichtskonstanz.

Man erhält 243 Teile N,N'-Diäthyl-N,N'-diphenyl--äthylendiamin in hellen Kristallen, die dünnschichtchromatographisch lediglich eine Spur N--Äthyl-N-(β-hydroxyäthyl)-anilin zeigen und einen Schmelzpunkt von 65 bis 70°C besitzen. Nach Umkristallisation aus Äthanol erhält man farblose Kristalle mit einem Fp. von 75°C, die chromatographisch einheitlich sind (nach Beilstein *12*, 545: Fp. 75°C).

*Beispiel 46*

214 Teile p-Toluidin, 150 Teile Äthylenglykol und 50 Teile einer 85%igen wässrigen Phosphorsäure werden zusammen etwa 12 Stunden bei einer Temperatur von 180 bis 190°C gerührt. In der Vorlage werden 90 Teile einer Wasser/Äthylenglykol-Mischung und 10 Teile p-Toluidin abgeschieden. Dünnschichtchromatographisch zeigt das Reaktionsprodukt als Verunreinigungen nur noch sehr geringe

Mengen von 3 Substanzen als Ausgangsprodukt und Nebenprodukte; man giesst das bei geringem Abkühlen kristallisierende Reaktionsprodukt noch heiss aus, zerkleinert den Kristallkuchen nach dem Erkalten und rührt ihn mit 1000 Teilen Wasser bei einem pH-Wert von 7 bis 7,5 (nach Zusatz von Natronlauge) drei Stunden lang aus. Man saugt sodann ab, wäscht den Rückstand mit Wasser und digeriert die leicht duktile Masse mit 500 Teilen Äthanol, wobei diese in helle Kristalle zerfällt. Man saugt diese ab, wäscht sie mit 100 Teilen Äthanol nach und trocknet sie bei 60°C.

Ausbeute: 220 Teile farblose Kristalle mit Smp. von 181 bis 189°C.

Nach Umkristallisation aus Äthanol erhält man die DC-einheitliche neue Verbindung N,N'-Bis-(4-methylphenyl)-piperazin mit einem Smp. von 189°C.

Analyse:

| | | | |
|---|---|---|---|
| ber.: | C 81,20% | gef.: | C 80,9% |
| | H 8,27% | | H 8,3% |
| | N 10,52% | | N 10,5% |
| | O 0,00% | | O 0,0% |

*Beispiel 47*

Ein Gemisch aus 214 Teilen m-Toluidin, 180 Teilen Äthylenglykol und 25 Teilen einer wässrigen 85%igen Phosphorsäure werden 12 Stunden lang bei 180 bis 190°C gerührt. In der Vorlage scheiden sich 100 Teile eines Wasser/Äthylenglykol-Gemisches und 10 Teile m-Toluidin ab. Man giesst das Reaktionsgemisch bei etwa 130°C unter Rühren in 1000 Teile Wasser und stellt mit Natronlauge auf einen pH-Wert von 7 bis 7,5. Die granuliert ausgefallene Kristallmasse wird abgesaugt, mit Wasser neutral gewaschen und der schwach duktile Filterrückstand in 500 Teilen Äthanol digeriert, wobei er zu einem farblosen Kristallbrei zerfällt. Man saugt ihn ab und trocknet ihn bei 60°C. Ausbeute: 220 Teile farblose Kristalle mit Smp. von 116 bis 122°C. Dünnschichtchromatographisch sind noch sehr geringe Mengen von drei anderen Produkten als Verunreinigungen nachweisbar.

Nach Umkristallisation aus Äthanol erhält man die DC-einheitliche, neue Verbindung N,N'-Bis-(3-methylphenyl)-piperazin mit einem Smp. von 124 bis 125°C.

Analyse:

| | | | |
|---|---|---|---|
| ber.: | C 81,20% | gef.: | C 80,9% |
| | H 8,27% | | H 8,2% |
| | N 10,52% | | N 10,7% |
| | O 0,00% | | O 0,0% |

*Beispiel 48*

Eine Mischung aus 350 Teilen 2,5-Dichloranilin, 62 Teilen Äthylenglykol und 25 Teilen einer wässrigen 85%igen Phosphorsäure wird 8 Stunden lang bei 180 bis 190°C gerührt. In der Destillationsvorlage werden 39 Teile Wasser und 2 Teile 2,5-Dichloranilin abgeschieden. Die heisse Reaktionsschmelze wird ausgegossen, nach dem Erkalten die erstarrte Masse zerschlagen und in einer alkalischen Lösung aus 1000 Teile kaltem Wasser und 40 Volumenteilen einer wässrigen 33%igen Natronlauge ausgerührt. Es wird dann abgesaugt, der Filterrückstand neutral gewaschen und in 1000 Teilen digeriert, wobei das Produkt zu hellen Kristallen zerfällt. Man saugt sie ab und trocknet sie bei 60°C. Ausbeute: 305 Teile, die neben dem Reaktionsprodukt nur Spuren an Ausgangsamin und 2 Nebenprodukte enthalten.

Nach Umlösen in Äthanol erhält man farblose Kristalle der neuen Verbindung N,N'-Bis-(2,5-dichlorphenyl)-äthylendiamin mit einem Smp. von 135 bis 139°C.

Analyse:

| | | | |
|---|---|---|---|
| ber.: | C 48,00% | gef.: | C 47,8% |
| | H 3,40% | | H 3,5% |
| | Cl 40,57% | | Cl 39,9% |
| | N 8,00% | | N 8,1% |
| | O 0,00% | | O 0,0% |

*Beispiel 49*

250 Teile m-Chloranilin, 136 Teile Äthylenglykol und 25 Teile einer wässrigen 85%igen Phosphorsäure werden zusammen 12 Stunden bei 180 bis 190°C gerührt. Während dieser Zeit scheiden sich 78 Teile eines Gemisches aus Wasser und Äthylenglykol und 3 Teile m-Chloranilin ab. Anschliessend wird der Reaktionsansatz auf 130°C abgekühlt und bei dieser Temperatur in eine alkalische Lösung aus 1000 Teilen kaltem Wasser 40 Volumenteilen einer wässrigen 33%igen Natronlauge eingerührt. Nicht umgesetztes m-Chloranilin und flüchtige Nebenprodukte werden mit Wasserdampf abgetrieben; nach Abtrennen der wässrigen Phase erhält man das halbfeste Reaktionsprodukt, das im Dünnschichtchromatogramm neben dem Hauptprodukt, der Piperazinverbindung, noch N,N'-Bis-(3-chlorphenyl)-äthylendiamin als Nebenprodukt enthält. Man digeriert es in 1000 Teilen Äthanol; hierbei bleiben helle Kristalle ungelöst zurück, die abgesaugt, gewaschen und getrocknet werden. Man erhält als Rückstand 150 Teile eines kristallinen Produktes, das nur wenig der genannten Äthylendiamin-Verbindung enthält.

Nach Umkristallisation aus Äthanol erhält man die neue, DC-einheitliche Verbindung N,N'-Bis-(3-chlorphenyl)-piperazin mit einem Smp. von 120 bis 122°C.

Analyse:

| | | | |
|---|---|---|---|
| ber.: | C 62,5% | gef.: | C 62,6% |
| | H 5,2% | | H 5,2% |
| | Cl 23,1% | | Cl 23,1% |
| | N 9,1% | | N 9,5% |
| | O 0,0% | | O 0,0% |

*Beispiel 50*

Eine Mischung aus 280 Teilen o-Chloranilin, 62 Teilen Äthylenglykol und 25 Teilen einer wässrigen 85%igen Phosphorsäure wird 8 Stunden lang bei einer Temperatur von 180 bis 190°C gerührt. 38 Teile Wasser und 7 Teile o-Chloranilin gehen hierbei destillativ über. Nach dieser Reaktion gibt man bei 100°C 10 Teile Magnesiumoxid in den Reaktionsansatz und saugt nach 30 Minuten über eine erwärmte Nutsche vom Magnesiumphosphat ab. Man arbeitet durch fraktionierte Destillation auf und erhält die gewünschte Äthylendiaminverbindung bei einer Sumpftemperatur von 200 bis 218°C und einer Kopftemperatur von 200 bis 203°C und einem Druck von 1,3 mbar.

Die neue, DC-einheitliche Verbindung, die in der Vorlage erstarrt, besitzt einen Smp. von 68°C. Ausbeute: 245 Teile.

Analyse:

| | | | |
|---|---|---|---|
| ber.: C | 59,78% | gef.: C | 59,7% |
| H | 4,98% | H | 4,9% |
| Cl | 25,26% | Cl | 25,2% |
| N | 9,96% | N | 10,1% |
| O | 0,0 % | O | 0,0% |

Die Analyse entspricht der Konstitution N,N'-Bis-(2-chlorphenyl)-äthylendiamin.

*Beispiel 51*

Ein Gemisch aus 230 Teilen o-Toluidin, 62 Teilen Äthylenglykol und 15 Teilen einer wässrigen 85%igen Phosphorsäure wird 10 Stunden lang bei 180 bis 190°C gerührt. 39 Teile Wasser und 5 Teile o-Toluidin gehen über. Anschliessend kühlt man auf etwa 100°C ab, gibt 10 Teile Magnesiumoxid hinzu, rührt noch 30 Minuten und saugt heiss ab. Es wird anschliessend fraktioniert destilliert.

Bei einer Sumpftemperatur von 189 bis 100°C und einer Kopftemperatur von 183 bis 189°C und bei etwa 1,5 mbar geht die neue Verbindung N,N'-Bis-(2-methylphenyl)-äthylendiamin über und erstarrt in fast farblosen Kristallen. Ausbeute: 214 Teile; DC-einheitlich; Smp.: 69 bis 71°C.

Analyse:

| | | | |
|---|---|---|---|
| ber.: C | 80,0 % | gef.: C | 80,0% |
| H | 8,33% | H | 8,3% |
| N | 11,67% | N | 12,0% |
| O | 0,00% | O | 0,0% |

*Beispiel 52*

Ein Gemisch aus 230 Teilen o-Toluidin, 76 Teilen Propan-1,2-diol und 25 Teilen einer wässrigen 85%igen Phosphorsäure wird etwa 8 Stunden bei 180 bis 185°C gerührt. 40 Teile Wasser und 2 Teile o-Toluidin gehen destillativ über. Man gibt bei 100°C 10 Teile Magnesiumoxid hinzu, rührt noch 30 Minuten lang und saugt heiss ab. Die filtrierte Reaktionsmasse wird fraktioniert destilliert.

Die neue Verbindung der Formel

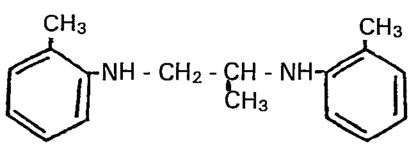

geht bei einer Siedetemperatur von 180 bis 182°C/1,5 mbar über. Ausbeute: 220 Teile eines schwach gelblichen Öles; DC-einheitlich.

Analyse:

| | | | |
|---|---|---|---|
| ber.: C | 80,31% | gef.: C | 80,4% |
| H | 8,61% | H | 8,7% |
| N | 11,02% | N | 11,0% |
| O | 0,00% | O | 0,0% |

*Beispiel 53*

Ein Gemisch aus 230 Teilen N-Methyl-anilin, 76 Teilen Propan-1,2-diol und 25 Teilen einer 85%igen wässrigen Phosphorsäure wird unter Rühren auf 180 bis 185°C erwärmt und bei dieser Temperatur noch 8 Stunden weitergerührt. Während der Reaktion gehen 40 Teile Wasser und 5 Teile N-Methylanilin über. Danach gibt man bei 80°C 10 Teile Magnesiumoxid hinzu, rührt 30 Minuten lang und saugt schliesslich ab. Das dickflüssige Öl wird anschliessend fraktioniert destilliert.

Bei einer Siedetemperatur von 180 bis 184°C/2,5 mbar erhält man die gewünschte, neue Verbindung N,N'-Diphenyl-N,N'-dimethyl-1,2-propylendiamin der Formel

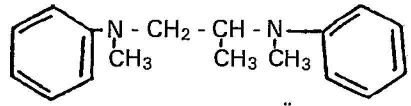

als schwach gelbes, dickflüssiges Öl. Ausbeute: 224 Teile.

Analyse:

| | | | |
|---|---|---|---|
| ber.: C | 80,31% | gef.: C | 80,0% |
| H | 8,66% | H | 8,6% |
| N | 11,02% | N | 11,0% |
| O | 0,00% | O | 0,0% |

*Beispiel 54*

Ein Gemisch aus 260 Teilen o-Anisidin, 76 Teilen Propan-1,2-diol und 15 Teilen einer 85%igen wässrigen Phosphorsäure wird bei einer Temperatur von 180 bis 185°C 8 Stunden lang gerührt. Während der Reaktion scheiden sich 38 Teile Wasser und 2 Teile o-Anisidin ab. Nach Abkühlen auf 120°C gibt man 10 Teile Magnesiumoxid hinzu und saugt nach halbstündigem Rühren über eine angewärmte Nutsche ab. Das geklärte Produkt wird sodann fraktioniert destilliert.

Bei einer Siedetemperatur von 196 bis 200°C/1,5 mbar erhält man 246 Teile der neuen Verbindung N,N'-Bis-(2'-methoxyphenyl)-1,2-propylendiamin, die in der Vorlage zu einer schwach gelblichen Kristallmasse erstarrt.

Aus Äthanol umkristallisiert zeigt sie einen Smp. von 91°C; sie ist DC-einheitlich.

Analyse:

| | | | |
|---|---|---|---|
| ber.: C | 71,33% | gef.: C | 71,2% |
| H | 7,69% | H | 7,8% |
| N | 9,79% | N | 10,1% |
| O | 11,19% | O | 11,8% |
| $OCH_3$ | 21,68% | $OCH_3$ | 21,9% |

*Beispiel 55*

Ein Gemisch aus 200 Teilen Anilin, 76 Teilen Propan-1,2-diol und 25 Teilen einer wässrigen 85%igen Phosphorsäure wird bei 180 bis 185°C 10 Stunden lang gerührt; 40 Teile Wasser und 3 Teile Anilin scheiden sich ab. Man kühlt sodann auf etwa 100°C ab, gibt 10 Teile Magnesiumoxid hinzu, rührt etwa 30 Minuten und saugt ab. Das filtrierte Produkt wird fraktioniert destilliert.

Bei 180 bis 188°C/1,5 mbar gehen 195 Teile eines gelblichen Öls über, das DC-einheitlich ist.

Die Analysenwerte

| | | | |
|---|---|---|---|
| ber.: C | 79,64% | gef.: C | 79,6% |
| H | 7,96% | H | 8,0% |
| N | 12,38% | N | 12,8% |
| O | 0,00% | O | 0,0% |

entsprechen dem N,N'-Bis-phenyl-1,2-propylendiamin.

*Beispiel 56*

Man rührt ein Gemisch aus 280 Teilen o-Phenetidin, 76 Teilen Propan-1,2-diol und 25 Teilen einer wässrigen 85%igen Phosphorsäure bei 180 bis 185°C während 10 Stunden, wobei 40 Teile Wasser und 2 Teile o-Phenetidin übergehen. Nach Abkühlen auf 120 bis 130°C gibt man 10 Teile Magnesiumoxid hinzu, rührt noch 30 Minuten und saugt schliesslich über eine angewärmte Nutsche ab. Das geklärte Filtrat wird fraktioniert destilliert. Das Hauptprodukt geht bei einer Siedetemperatur von 200 - 210°C/ 1,5 - 1,7 mbar über und erstarrt in der Vorlage kristallin. Ausbeute: 270 Teile.

Aus Äthanol umgelöst erhält man DC-einheitlich die Verbindung N,N'-Bis-(2-äthoxyphenyl)-1,2-propylendiamin mit einem Smp. von 67 bis 68°C.

*Beispiel 57*

Man rührt bei 180 bis 190°C während 8 Stunden ein Gemisch aus 260 Teilen o-Anisidin, 90 Teilen Butan-1,2-diol und 25 Teilen einer wässrigen 85%-igen Phosphorsäure; 40 Teile Wasser und 2 Teile o-Anisidin gehen während der Reaktion über. Bei etwa 120°C setzt man anschliessend 10 Teile Magnesiumoxid hinzu, rührt noch 30 Minuten und saugt ab. Das filtrierte Produkt wird fraktioniert destilliert. Bei einer Sumpftemperatur von 215 bis 230°C und einer Kopftemperatur von 205 bis 215°C bei einem Druck von 1,5 mbar gehen 260 Teile eines farblosen Produktes über, das in der Vorlage erstarrt.

Nach Umkristallisation in Äthanol erhält man die DC-einheitliche neue Verbindung N,N'-Bis-(2'--methoxyphenyl)-1,2-butylendiamin der Formel

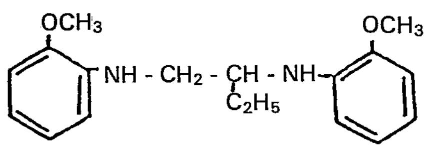

mit einem Smp. von 68 bis 70°C.

Analyse:

| | | | | |
|---|---|---|---|---|
| ber.: | C | 72,0% | gef.: C | 72,2& |
| | H | 8,0% | H | 7,9% |
| | N | 9,3% | N | 9,3% |
| | O | 10,6% | O | 10,8% |
| | $OCH_3$ | 20,6% | $OCH_3$ | 20,5% |

*Beispiel 58*

Ein Gemisch aus 450 Teilen N-Methyl-anilin, 180 Teilen Butan-1,2-diol und 50 Teilen einer 85%igen wässrigen Phosphorsäure wird bei 180 bis 190°C 10 Stunden lang gerührt; während diesen Zeit gehen 78 Teile Wasser und 20 Teile N-Methyl-anilin über. Nach Abkühlen auf 80°C gibt man 20 Teile Magnesiumoxid hinzu, rührt etwa 30 Minuten weiter, saugt ab und fraktioniert anschliessend das Filtrat durch Destillation. Bei einer Siedetemperatur von 184 bis 186°C/2,3 mbar gehen 450 Teile eines gelblichen dickflüssigen Öls über, das DC-einheitlich ist. Die Analyse entspricht der neuen Verbindung der Formel

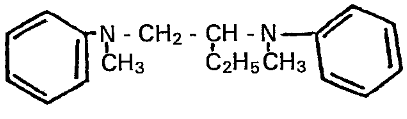

Analyse:

| | | | | |
|---|---|---|---|---|
| ber.: | C | 80,60% | gef.: C | 80,6% |
| | H | 8,96% | H | 8,9% |
| | N | 10,45% | N | 10,8% |
| | O | 0,00% | O | 0,0% |

*Beispiel 59*

Man verfährt wie im Beispiel 39 beschrieben, ersetzt jedoch die Dihydroxyäthyl-anilin-Verbindung durch eine äquivalente Menge an N,N-Di-(β-hydroxyäthyl)-3-chloranilin; man erhält in gleich guter Ausbeute und Reinheit die Verbindung der Formel

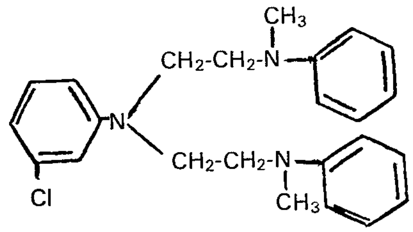

*Beispiel 60*

428 Teile N-Methylanilin und 25 Teile einer 85%-igen wässrigen Phosphorsäure werden unter Rühren auf 90 bis 95°C erwärmt. Man verdrängt die Luft durch Stickstoff und gibt stetig innerhalb von 8 Stunden 145 Teile n-Butylenoxid-(1,2) hinzu. Man rührt noch 2 Stunden nach und erhitzt sodann den Reaktionsansatz innerhalb einer Stunde auf 185 bis 190°C und hält diese Temperatur etwa 10 Stunden. Hierbei destillieren 38 Teile Wasser und 10 Teile N-Methylanilin ab. Man kühlt den Ansatz sodann auf etwa 100°C ab, gibt 20 Teile Magnesiumoxid hinzu und saugt nach 30 Minuten über eine vorgewärmte Nutsche ab. Das erhaltene dickflüssige Öl wird fraktioniert destilliert; bei einer Siedetemperatur von 184 bis 186°C und einem Druck von 2 mbar gehen 430 Teile N,N'-Dimethyl-N,N'-diphenyl-1,2-n-butylendiamin über. Das Produkt gleicht demjenigen von Beispiel 58.

*Beispiel 61*

321 Teile N-Methylanilin und 25 Teile einer 85%-igen wässrigen Phosphorsäure werden gemischt und unter Rühren auf 90°C erhitzt. Sodann gibt man stetig unter die Oberfläche des Reaktionsansatzes im Verlauf von 8 Stunden 90 Teile Propylenoxid hinzu. Während der Reaktion lässt man einen schwachen Stickstoffstrom durch das Reaktionsgefäss streichen. Man rührt noch etwa 2 Stunden bei 90°C nach und steigert sodann innerhalb von 2 Stunden die Temperatur auf 180 bis 190°C. Die Reaktion wird etwa 8 Stunden bei dieser Temperatur weitergeführt, innerhalb der sich 30 Teile Wasser und 3 Teile N-Methylanilin abscheiden. Man kühlt sodann auf etwa 80°C ab, gibt verdünnte wässrige Natronlauge bis zur schwach alkalischen Reaktion hinzu und unterwirft den Ansatz einer Wasserdampfdestillation, bis kein Öl mehr übergeht. Der Destillationsrückstand wird sodann zweimal mit Wasser gewaschen. Nach Abtrennung der Wasserphase erhält man etwa 340 Teile eines schwach bräunlichen, dickflüssigen Öls aus N,N'-Diphenyl-N,N'-dimethyl-1,2-propylendiamin, das durch fraktionierte Destillation, beispielsweise bei einem Siedepunkt von 179 bis

183°C/2 mbar, rein gewonnen wird. Das Produkt gleicht dem von Beispiel 53.

*Beispiel 62*

230 Teile o-Toluidin, 62 Teile Äthylenglykol und 20 Teile kristallisierte phosphorige Säure werden gemischt und sodann 8 Stunden bei einer Temperatur von 180 bis 190°C gerührt. In einer Destillationsvorlage scheiden sich 35 Teile Wasser und 5 Teile o-Toluidin ab. Man kühlt den Ansatz auf 80°C, gibt verdünnte wässrige Natronlauge bis zur schwach alkalischen Reaktion hinzu und unterwirft ihn sodann einer Wasserdampfdestillation, bis kein Öl mehr übergeht. Anschliessend rührt man ihn in 2000 Teile kaltes Wasser und saugt das granulierte Produkt ab. Man trocknet es bis zur Gewichtskonstanz und erhält 220 Teile schwach rötlicher Kristalle der Verbindung N,N'-Bis-(2-methyl-phenyl)-äthylendiamin, die dem Reaktionsprodukt von Beispiel 51 gleicht.

*Beispiel 63*

165 Teile N-Äthyl-N'-(β-hydroxyäthyl)-anilin, 127 Teile N-Äthylanilin und 40 Teile einer 50%igen wässrigen phosphorigen Säure werden gemischt und im Verlauf von etwa 1 Stunde auf 180 bis 185°C erhitzt; der Reaktionsansatz wird bei dieser Temperatur noch etwa 8 Stunden weitergerührt. Während des Reaktionsumsatzes, beginnend bei etwa 140°C, scheiden sich 38 Teile Wasser und 2 bis 3 Teile einer Mischung der beiden Ausgangsaminoverbindungen ab (die in einer Destillationsvorlage aufgefangen werden können). Das Ende der Reaktion lässt sich dünnschichtchromatographisch feststellen. Man kühlt danach den Reaktionsansatz auf etwa 80°C ab, stellt ihn mit wässriger Natronlauge schwach alkalisch und unterwirft ihn einer Wasserdampfdestillation, bis kein Öl mehr übergeht. Er wird anschliessend in 2000 Teile kaltes Wasser eingerührt, wobei sich das gebildete Reaktionsprodukt in hellen, schwach grau gefärbten Granalien abscheidet. Man saugt sie ab, wäscht sie mit Wasser neutral und trocknet sie bis zur Gewichtskonstanz. Es werden 238 Teile N,N'-Diäthyl-N,N'-diphenyl-äthylendiamin erhalten, das dem Produkt des Beispiels 3 gleicht.

*Beispiele 64 bis 71*

Man verfährt in erfindungsgemässer Weise, beispielsweise analog einer der in den obigen Ausführungsbeispielen beschriebenen Verfahrensweise, indem man von den jeweiligen, in den nachstehenden Tabellenbeispielen angegebenen Ausgangsverbindungen ausgeht und die saure Kondensationsreaktion bei einer Temperatur von etwa 180 bis 190°C mittels phosphoriger Säure als saurem Kondensationsmittel durchführt. Man erhält erfindungsgemäss die in der nachfolgenden Tabelle angegebenen Äthylendiamin-Endprodukte entsprechend der allgemeinen Formel (1) aus den nachfolgend in einer gesonderten Tabelle angegebenen, eingesetzten Ausgangsverbindungen.

| Bsp. | Verbindung der Formel (1) |
|---|---|
| 64 | 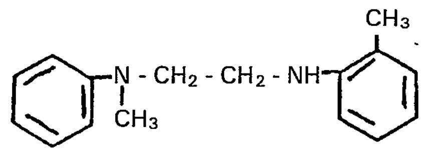 |
| 65 | 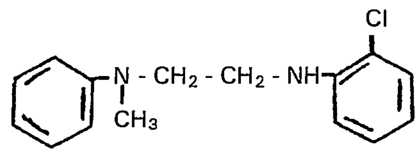 |
| 66 | 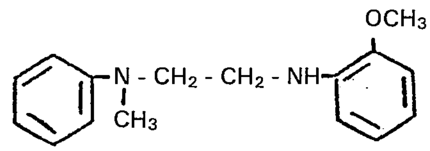 |
| 67 | 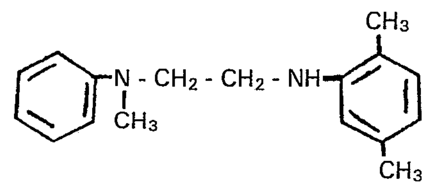 |
| 68 | 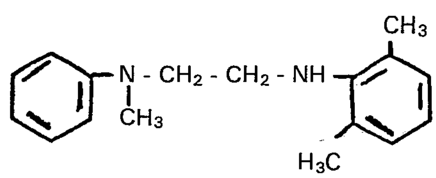 |
| 69 | 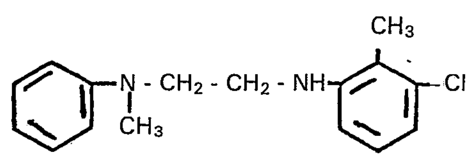 |
| 70 | 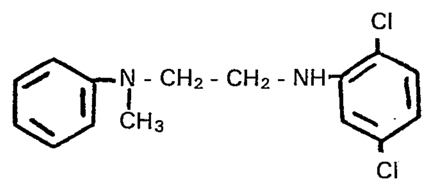 |
| 71 | 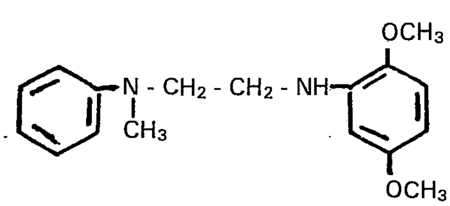 |

| Bsp. | hergestellt aus äquimolaren Mengen der Verbindung (3) und der Verbindung (4) | | Kp. |
|---|---|---|---|
| 64 | N-Methyl-N-(β-hydroxy-äthyl)-anilin | 2-Methyl-anilin | 179°C/2 mbar |
| 65 | dito | 2-Chloranilin | 190°C/1,6 mbar |
| 66 | dito | 2-Methoxy-anilin | 210°C/1,3 mbar |
| 67 | dito | 2,5-Dimethyl-anilin | 190°C/1,2 mbar |
| 68 | dito | 2,6-Dimethyl-anilin | 175°C/1,6 mbar |
| 69 | N-Methyl-N-(β-hydroxy-äthyl)-anilin | 3-Chlor-2-methyl-anilin | 190°C/2 mbar |
| 70 | dito | 2,5-Dichlor-anilin | 200°C/1,3 mbar |
| 71 | dito | 2,5-Dimethoxy-anilin | > 225°C/1,3 mbar |

**Patentansprüche**

1. Verfahren zur Herstellung von N,N'-Diarylalkylen-diaminen und N,N',N''-Triaryl-dialkylen-triaminen der allgemeinen Formel (1)

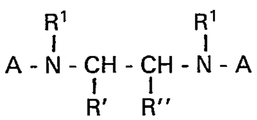

(1)

in welcher

beide A zueinander gleiche oder voneinander verschiedene Bedeutungen haben und jedes einen gegebenenfalls substituierten Arylrest bedeutet,

R' und R'' zueinander gleiche oder voneinander verschiedene Bedeutungen haben und jedes ein Wasserstoffatom oder eine niedere Alkylgruppe ist,

beide $R^1$ zueinander gleiche oder voneinander verschiedene Bedeutungen haben und jedes ein Wasserstoffatom oder ein gegebenenfalls substituierter Alkyl-, Aralkyl-, Aryl- oder Cycloalkylrest bedeutet oder eines der $R^1$ eine Gruppe der allgemeinen Formel (2)

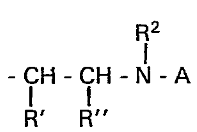

(2)

ist, in welcher

R', R'' und A die obengenannte Bedeutung haben und

$R^2$ ein Wasserstoffatom oder ein gegebenenfalls substituierter Alkyl-, Aralkyl-, Aryl- oder Cycloalkylrest ist,

wobei $R^2$ in Formel (2) die gleiche oder eine davon verschiedene Bedeutung von $R^1$ von Formel (1) und A zu den beiden A in Formel (1) jeweils gleiche oder verschiedene Bedeutungen besitzen können,

oder worin,

beide $R^1$ zusammen vereinigt einen Rest der Formel

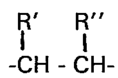 bzw. 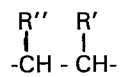

mit R' und R'' der obigen Bedeutung darstellen, bei welchem man

a) ein β-Hydroxyalkyl-arylamin der allgemeinen Formel (3)

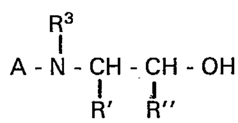

(3)

in welcher A, R' und R'' die obengenannten Bedeutungen haben und $R^3$ für einen gegebenenfalls substituierten Alkyl-, Aralkyl-, Aryl- oder Cycloalkylrest steht, mit einem Amin der allgemeinen Formel (4)

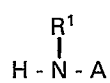

(4)

in welcher A und $R^1$ die obengenannten Bedeutungen haben, zur Verbindung (1), in welcher beide A sowie R' und R'' die obengenannten Bedeutungen haben und das eine $R^1$ ein gegebenenfalls substituierter Alkyl-, Aralkyl-, Aryl- oder Cycloalkylrest ist und das andere $R^1$ ein Wasserstoffatom oder ein gegebenenfalls substituierter Alkyl-, Aralkyl-, Aryl- oder Cycloalkylrest ist, umsetzt oder

b) ein β-Hydroxyalkyl-arylamin der allgemeinen Formel (3a)

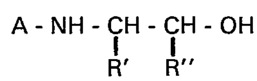

(3a)

in welcher A, R' und R'' die obengenannten Bedeutungen haben, mit einem β-Hydroxyalkyl-arylamin der allgemeinen Formel (3b)

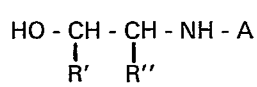

(3b)

in welcher A, R' und R'' die obengenannten Bedeutungen haben, zur Verbindung der Formel (6a) bzw. (6b)

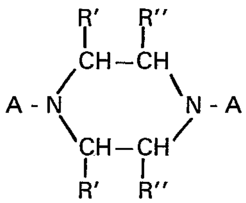
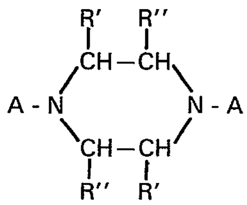

(6a) (6b)

in welchen beide A sowie R' und R'' die obengenannten Bedeutungen haben, umsetzt oder

c) eine Alkylenglykol-Verbindung der allgemeinen Formel (5)

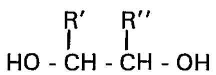
(5)

in welcher R' und R'' die obengenannten Bedeutungen haben, oder dessen Alkylenoxid mit einem Amin der allgemeinen Formel (4)

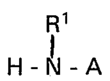
(4)

in welcher $R^1$ und A die obengenannten Bedeutungen haben, oder mit zwei verschiedenen Aminen der Formel (4), in welchen A oder $R^1$ oder beide verschiedene Bedeutungen haben, zur Verbindung (1), in welcher beide A sowie R' und R'' die obengenannten Bedeutungen haben und beide $R^1$, beide gleich oder verschieden voneinander, jeweils für ein Wasserstoffatom oder einen gegebenenfalls substituierten Alkyl-, Aralkyl-, Aryl- oder Cycloalkylrest stehen, umsetzt oder

d) ein Amin der obigen Formel (3), in welcher $R^3$ für eine Gruppe der oben definierten Formel (2) steht und A, R' und R'' die oben genannten Bedeutungen haben, mit einem Amin der obigen Formel (4), in welcher $R^1$ für ein Wasserstoffatom steht und A die obengenannte Bedeutung besitzt, zur Verbindung (1), in welcher beide A sowie R' und R'' die obengenannten Bedeutungen haben, das eine $R^1$ für die oben definierte Gruppe der Formel (2) steht und das andere $R^1$ ein Wasserstoffatom bedeutet, umsetzt, oder

e) ein Amin der obigen Formel (3), in welcher $R^3$ für eine Gruppe der Formel -CH(R')-CH(R'')-OH steht und A, R' und R'' die obengenannten Bedeutungen haben, mit einem Amin der obigen Formel (4), in welcher A und $R^1$ die obengenannten Bedeutungen haben, zur Verbindung (1), in welcher beide A sowie R', R'' und das eine $R^1$ die obengenannten Bedeutungen haben und das andere $R^1$ für einen Rest der Formel (2) steht, umsetzt oder

f) ein Amin der obigen Formel (3), in welcher A, $R^3$, R' und R'' die obengenannten Bedeutungen haben, mit einem Amin der allgemeinen Formel (4), in welcher A die obengenannte Bedeutung besitzt und $R^1$ für ein Wasserstoffatom steht, zur Verbindung (1), in welcher beide A sowie R', R'' und das eine $R^1$ die hier genannten Bedeutungen haben und das andere $R^1$ für einen Rest der Formel (2) steht, umsetzt oder

g) eine Verbindung der Formel (4), in welcher A die obengenannte Bedeutung besitzt und $R^1$ für ein Wasserstoffatom steht, mit einer Verbindung der Formel (5), in welcher R' und R'' die obengenannten Bedeutungen haben, zur Verbindung der oben definierten Formel (6a) bzw. (6b), in welchen beide A sowie R' und R'' die obengenannten Bedeutungen besitzen, umsetzt oder

h) eine Verbindung der obigen Formel (3), in welcher $R^3$ für eine Gruppe der Formel -CH(R')-CH(R'')-OH steht und A, R' und R'' die obengenannten Bedeutungen haben, mit einer Verbindung der Formel (4), in welcher A die obengenannte Bedeutung besitzt und $R^1$ ein Wasserstoffatom ist, zur Verbindung der oben definierten Formel (6a) bzw. (6b) umsetzt,

dadurch gekennzeichnet, dass man die Umsetzung der Verbindungen in Gegenwart von Phosphorsäure oder von phosphoriger Säure in einer Menge von mindestens 0,01 Mol pro Mol Verbindung (3) oder (5) und bei einer Temperatur zwischen 150 und 225°C durchführt.

2. Verfahren nach Anspruch 1, dadurch gekennzeichnet, dass man die Reaktion bei einer Temperatur zwischen 170 und 200°C durchführt.

3. Verfahren nach Anspruch 1 oder 2, dadurch gekennzeichnet, dass man die Reaktion in Gegenwart einer Menge von 0,01 bis 0,3 Mol Phosphorsäure oder phosphoriger Säure pro Mol der Verbindung der Formel (3) oder (5) durchführt.

4. Verfahren nach Anspruch 1, 2 oder 3, bei welchem man eine Verbindung der allgemeinen Formel (3c) mit einem Amin der allgemeinen Formel (4a)

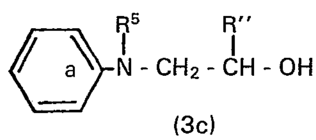
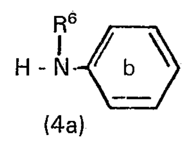

(3c) (4a)

in welchen die Benzolkerne a und b als substituierte oder unsubstituierte Phenylreste zueinander gleich oder verschieden voneinander sein können, R'' für ein Wasserstoffatom, die Methyl- oder Äthylgruppe steht, $R^5$ und $R^6$ gleich oder verschieden voneinander sind und $R^5$ einen gegebenenfalls substituierten Alkyl-, Aralkyl-, Aryl- oder Cycloalkylrest bedeutet und $R^6$ für ein Wasserstoffatom oder einen gegebenenfalls substituierten Alkyl-, Aralkyl-, Aryl-, oder Cycloalkylrest steht, zu einer Verbindung der allgemeinen Formel (7)

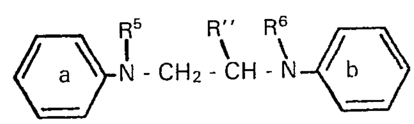
(7)

in welcher die Benzolkerne a und b und R'', $R^5$ und $R^6$ die obengenannten Bedeutungen haben, umsetzt.

5. Verfahren nach Anspruch 1, 2 oder 3, bei welchem man eine Verbindung der allgemeinen Formel (5) mit einem Amin der allgemeinen Formel (4a)

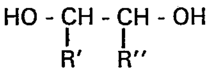

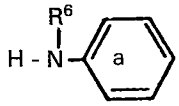

(5) (4a)

in welcher der Benzolkern a ein substituierter oder unsubstituierter Phenylrest bedeutet, $R^6$ ein gegebenenfalls substituierter Alkyl-, Aralkyl-, Aryl- oder Cycloalkylrest ist und R' und R'' die in Anspruch 1 genannten Bedeutungen besitzen, zu einer Verbindung der allgemeinen Formel (8)

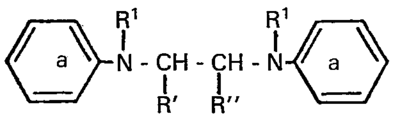

(8)

in welcher die Benzolkerne a als substituierte oder unsubstituierte Phenylreste zueinander gleich sind und $R^1$, jedes gleich zueinander, ein gegebenenfalls substituierter Alkyl-, Aralkyl-, Aryl- oder Cycloalkylrest ist, umsetzt.

6. Verfahren nach einem der Ansprüche 1, 2 oder 3, bei welchem man eine Verbindung der allgemeinen Formel

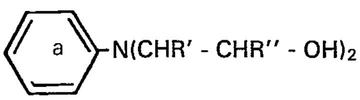

mit einer Verbindung der allgemeinen Formel

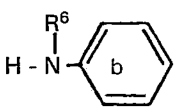

in welchen die Benzolkerne a und b und der Rest $R^6$ die in Anspruch 5 genannte Bedeutung haben, $R^6$ jedoch zwingend nicht Wasserstoff ist, und R' und R'' die in Anspruch 1 genannten Bedeutungen besitzen, bevorzugt beide, insbesondere R', für Wasserstoff stehen, zu einer Verbindung der allgemeinen Formel

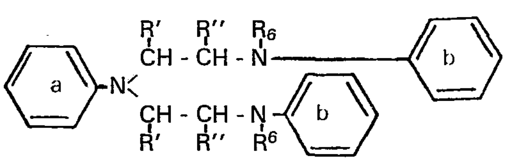

in welcher a, b, R', R'' und $R^6$ die eben genannten Bedeutungen haben, umsetzt.

7. Verfahren nach Anspruch 1, 2 oder 3, bei welchem man eine Verbindung der allgemeinen Formel

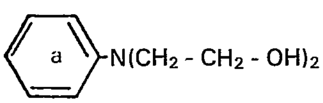

mit einer Verbindung der allgemeinen Formel

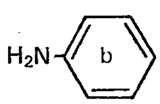

in welchen die Benzolkerne a und b als substituierte oder unsubstituierte Phenylreste zueinander gleich oder verschieden voneinander sein können, zu einer Verbindung der allgemeinen Formel

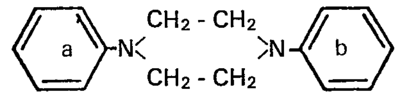

mit a und b der genannten Bedeutung umsetzt.

**Revendications**

1. Procédé de préparation de N,N'-diaryl-alkylène-diamines et de N,N',N''-triaryl-dialkylène-triamines répondant à la formule générale (1):

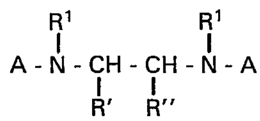

(1)

dans laquelle

les deux A représentent chacun, indépendamment l'un de l'autre, un radical aryle, éventuellement substitué,

R' et R'' représentent chacun, indépendamment l'un de l'autre, un atome d'hydrogène ou un radical alkyle inférieur,

les deux $R^1$ représentent chacun, indépendamment l'un de l'autre, un atome d'hydrogène ou un radical alkyle, aralkyle, aryle ou cycloalkyle, éventuellement substitué, l'un des $R^1$ pouvant représenter aussi un radical répondant à la formule générale (2):

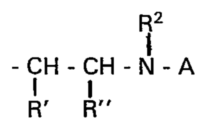

(2)

dans laquelle

R', R'' et A ont les significations indiquées ci-dessus et

$R^2$ représente un atome d'hydrogène ou un radical alkyle, aralkyle, aryle ou cycloalkyle éventuellement substitué,

le symbole $R^2$ présent dans la formule (2) pouvant avoir la même signification que $R^1$ dans la formule (1) ou une signification différente et A pouvant avoir une signification identique à celles des deux A présents dans la formule (1) ou une signification différente, ou

les deux $R^1$ sont unis l'un à l'autre et représentent un radical répondant à l'une des formules:

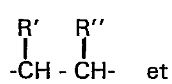 et 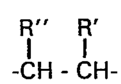

dans lesquelles R' et R'' ont les significations précédemment données,

procédé selon lequel:

a) on fait réagir une $\beta$-hydroxyalkyl-arylamine répondant à la formule générale (3):

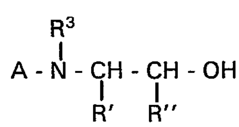

(3)

dans laquelle A, R′ et R′′ ont les significations précédemment données et $R^3$ représente un radical alkyle. aralkyle, aryle ou cycloalkyle éventuellement substitué, avec une amine répondant à la formule générale (4):

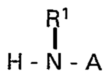

(4)

dans laquelle A et $R^1$ ont les significations précédemment données, réaction qui conduit à un composé (1) dans lequel les deux A ainsi que R′ et R′′ ont les significations indiquées plus haut, l'un des $R^1$ représente un radical alkyle, aralkyle, aryle ou cycloalkyle éventuellement substitué et l'autre $R^1$ représente un atome d'hydrogène ou un radical alkyle, aralkyle, aryle ou cycloalkyle éventuellemen substitué, ou

b) on fait réagir une $\beta$-hydroxyalkyl-arylamine répondant à la formule générale (3a):

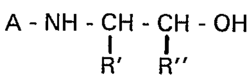

(3a)

dans laquelle A, R′ et R′′ ont les significations précédemment données, avec une $\beta$-hydroxyalkyl-arylamine répondant à la formule générale (3b):

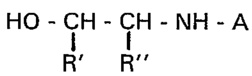

(3b)

dans laquelle A, R′ et R′′ ont les significations précédemment données, réaction qui conduit à un composé répondant à l'une des formules (6a) et (6b):

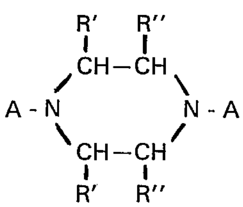

(6a)

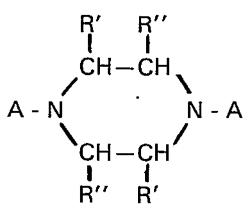

(6b)

dans lesquelles les deux A ainsi que R′ et R′′ ont les significations indiquées plus haut, ou

c) on fait réagir un alkylène-glycol répondant à la formule générale (5):

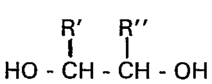

(5)

dans laquelle R′ et R′′ ont les significations précédemment données, ou l'oxyde d'alkylène qui en dérive, avec une amine répondant à la formule générale (4):

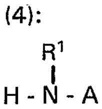

(4)

dans laquelle $R^1$ et A ont les significations précédemment données, ou avec deux amines différentes de formule (4) dans lesquelles A ou $R^1$ ou les deux ont des significations différentes, de manière à obtenir un composé (1) dans lequel les deux A ainsi que R′ et R′′ ont les significations indiquées plus haut et les deux $R^1$, identiques l'un à l'autre ou différents l'un de l'autre, représentent chacun un atome d'hydrogène ou un radical alkyle, aralkyle, aryle ou cycloalkyle éventuellement substitué, ou

d) on fait réagir une amine de formule (3) (voir ci-dessus) dans laquelle $R^3$ représente un radical répondant à la formule (2) définie ci-dessus et A, R′ et R′′ ont les significations précédemment données, avec une amine répondant à la formule (4) (voir ci-dessus) dans laquelle $R^1$ représente un atome d'hydrogène et A a la signification indiquée plus haut, réaction qui conduit à un composé (1) dans lequel les deux A ainsi que R′ et R′′ ont les significations précédemment données, l'un des $R^1$ désigne un radical de formule (2) tel que défini ci-dessus et l'autre $R^1$ représente un atome d'hydrogène, ou

e) on fait réagir une amine de formule (3) (voir ci-dessus) dans laquelle $R^3$ représente un radical -CH(R′)-CH(R′′)-OH et A, R′ et R′′ ont les significations précédemment données, avec une amine de formule (4) (voir ci-dessus) dans laquelle A et $R^1$ ont les significations indiquées ci-dessus, de manière à obtenir un composé (1) dans lequel les deux A ainsi que R′, R′′ et l'un des $R^1$ ont les significations précédemment données tandis que l'autre $R^1$ représente un radical de formule (2), ou

f) on fait réagir une amine de formule (3) (voir ci-dessus) dans laquelle A, $R^3$, R′ et R′′ ont les significations précédemment données, avec une amine de formule générale (4) dans laquelle A a la signification indiquée ci-dessus et $R^1$ représente un atome d'hydrogène, de manière à obtenir un composé (1) dans lequel les deux A ainsi que R′, R′′ et l'un des $R^1$ ont les significations qui viennent d'être données tandis que l'autre $R^1$ représente un radical de formule (2), ou

g) on fait réagir un composé de formule (4) dans lequel A a la signification précédemment donnée et $R^1$ représente un atome d'hydrogène, avec un composé de formule (5) dans lequel R′ et R′′ ont les significations précédemment données, de manière à obtenir un composé répondant à l'une des formules (6a) et (6b) précédemment définies dans lesquelles les deux A et R′ et R′′ ont les significations indiquées plus haut, ou

h) on fait réagir un composé de formule (3) (voir ci-dessus) dans lequel $R^3$ représente un radical -CH(R′)-CH(R′′)-OH et A, R′ et R′′ ont les significations précédemment données, avec un composé de formule (4) dans lequel A a la signification indiquée plus haut et $R^1$ représente un atome d'hydrogène, réaction qui conduit à un composé répondant à l'une des formules (6a) et (6b) définies ci-dessus,

procédé caractérisé en ce qu'on effectue la réaction des composés en présence d'acide phosphorique ou d'acide phosphoreux en une quantité d'au moins 0,01 mole par mole de composé (3) ou (5) et à une température comprise entre 150 et 225°C.

2. Procédé selon la revendication 1, caractérisé en ce qu'on effectue la réaction à une température comprise entre 170 et 200°C.

3. Procédé selon l'une des revendications 1 et 2, caractérisé en ce qu'on effectue la réaction en présence d'une quantité de 0,01 à 0,3 mole d'acide phosphorique ou d'acide phosphoreux par mole du composé de formule 3 ou 5.

4. Procédé selon l'une quelconque des revendications 1, 2 et 3 selon lequel on fait réagir un composé de formule générale 3c avec une amine de formule générale 4a

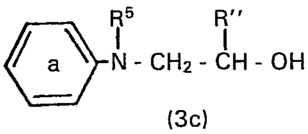

(3c)

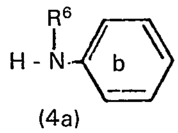

(4a)

formules dans lesquelles les noyaux benzéniques a et b, en tant que radicaux phényles substitués ou non, peuvent être identiques l'un à l'autre ou différents l'un de l'autre, R'' représente un atome d'hydrogène ou un radical méthyle ou éthyle, et $R^5$ et $R^6$ sont identiques l'un à l'autre ou différents l'un de l'autre, $R^5$ représentant un radical alkyle, aralkyle, aryle ou cycloalkyle éventuellement substitué et $R^6$ un atome d'hydrogène ou un radical alkyle, aralkyle, aryle ou cycloalkyle éventuellement substitué, réaction qui conduit à un composé répondant à la formule générale (7):

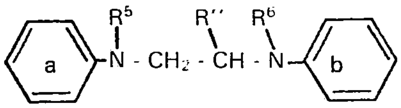

(7)

dans laquelle les noyaux benzéniques a et b, R'', $R^5$ et $R^6$ ont les significations indiquées ci-dessus.

5. Procédé selon l'une quelconque des revendications 1, 2 et 3 selon lequel on fait réagir un composé de formule générale (5) avec une amine de formule générale (4a):

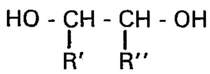

(5)

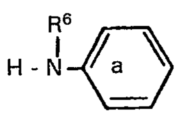

(4a)

formules dans lesquelles a représente un radical phényle substitué ou non, $R^6$ représente un radical alkyle, aralkyle, aryle ou cycloalkyle éventuellement substitué, et R' et R'' ont les significations données à la revendication 1, réaction qui conduit à un composé répondant à la formule générale (8);

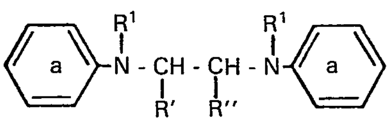

(8)

dans laquelle les noyaux benzéniques a, en tant que radicaux phényles substitués ou non, sont identiques l'un à l'autre et les $R^1$ sont identiques l'un à l'autre et représentent chacun un radical alkyle, aralkyle, aryle ou cycloalkyle éventuellement substitué.

6. Procédé selon l'une quelconque des revendications 1, 2 et 3, selon lequel on fait réagir un composé de formule générale

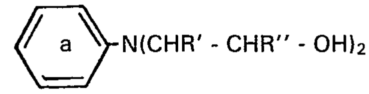

avec un composé de formule générale

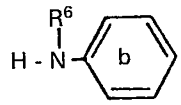

formules dans lesquelles les noyaux benzéniques a et b et le radical $R^6$ ont les significations données à la revendication 5 mais $R^6$ ne peut pas représenter l'hydrogène et R' et R'' ont les significations données à la revendication 1 et représentent de préférence tous les deux, surtout R', l'hydrogène réaction qui conduit à un composé répondant à la formule générale:

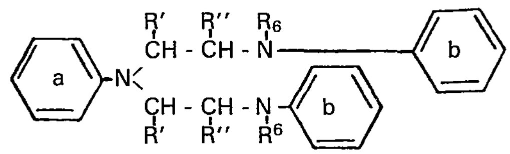

dans laquelle a, b, R', R'' et $R^6$ ont les significations qui viennent d'être indiquées.

7. Procédé selon l'une quelconque des revendications 1, 2 et 3 selon lequel on fait réagir un composé de formule générale:

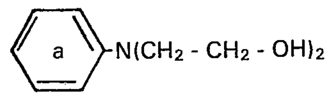

avec un composé de formule générale:

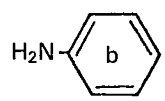

formules dans lesquelles les noyaux benzéniques a et b, en tant que radicaux phényles substitués ou non, sont identiques l'un à l'autre ou différents l'un de l'autre, réaction qui conduit à un composé répondant à la formule générale:

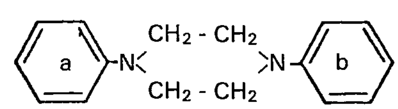

dans laquelle a et b ont les significations qui ont été données ci-dessus.

**Claims**

1. Process for the preparation of N,N'-diaryl-alkylene-diamines and N,N',N''-triaryl-dialkylene-triamines of the general formula (1)

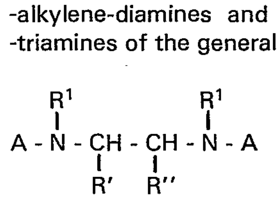

(1)

in which

both A have meanings which are identical to one another or different from one another, and each is an optionally substituted aryl radical,

R' and R'' have meanings which are identical to one another or different from one another, and each is a hydrogen atom or a lower alkyl group,

both $R^1$ have meanings which are identical to one another or different from one another, and each is a hydrogen atom or an optionally substituted alkyl, aralkyl, aryl or cycloalkyl radical, or one of the $R^1$ is a group of the general formula (2)

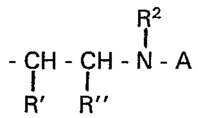 (2)

in which

R', R'' and A have the abovementioned meaning and

$R^2$ is a hydrogen atom or an optionally substituted alkyl, aralkyl, aryl or cycloalkyl radical,

$R^2$ having in formule (2) the same meaning as $R^1$ of formula (1) or having a meaning which is different from the meaning of $R^1$ in formula (1), and A having the same meaning to or a different meaning from both A in formula (1),

or wherein

both $R^1$ combined together represent a radical of the formula

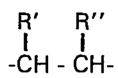 or 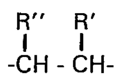

in which R' and R'' have the above meaning, in which process

a) a $\beta$-hydroxyalkyl-arylamine of the general formula (3)

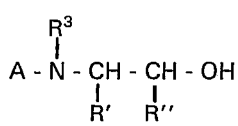 (3)

in which A, R' and R'' have the abovementioned meanings and $R^3$ is an optionally substituted alkyl, aralkyl, aryl or cycloalkyl radical, is reacted with an amine of the general formula (4)

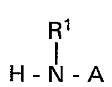 (4)

in which A and $R^1$ have the abovementioned meanings, to form the compound (1) in which both A as well as R' and R'' have the abovementioned meanings and one of $R^1$ is an optionally substituted alkyl, aralkyl, aryl or cycloalkyl radical and the other $R^1$ is a hydrogen atom or an optionally substituted alkyl, aralkyl, aryl or cycloalkyl radical, or

b) a $\beta$-hydroxyalkyl-arylamine of the general formula (3a)

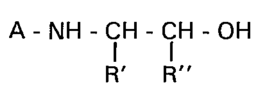 (3a)

in which A, R' and R'' have the abovementioned meanings, is reacted with a $\beta$-hydroxylalkyl-arylamine of the general formula (3b)

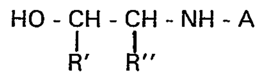 (3b)

in which A, R' and R'' have the abovementioned meanings, to form a compound of the formula (6a) or (6b)

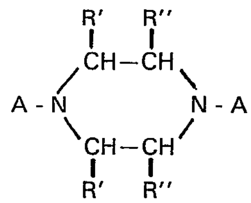 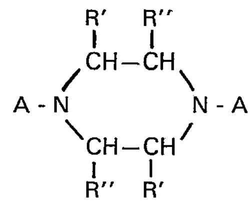

(6a) (6b)

in which both A as well as R' and R'' have the abovementioned meanings, or

c) an alkylene glycol compound of the general formula (5)

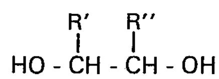 (5)

in which R' and R'' have the abovementioned meanings, or an alkyleneoxide thereof is reacted with an amine of the general formula (4)

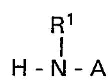 (4)

in which $R^1$ and A have the abovementioned meanings, or with two different amines of the formula (4), in which A or $R^1$ or both have different meanings, to form a compound (1) in which both A as well as R' and R'' have the abovementioned meanings and both $R^1$, being identical to or different from one another, each represents a hydrogen atom or an optionally substituted alkyl, aralkyl, aryl or cycloalkyl radical, or

d) an amine of the above formula (3), in which $R^3$ represents a group of the above-defined formula (2) and A, R' and R'' have the abovementioned meanings, is reacted with an amine of the above formula (4) in which $R^1$ is a hydrogen atom and A has the abovementioned meaning, to form a compound (1) in which both A as well as R' and R'' have the abovementioned meanings, one of the $R^1$ represents the above-defined group of the formula (2) and the other $R^1$ is a hydrogen atom, or

e) an amine of the above formula (3) in which $R^3$ is a group of the formula -CH(R')-CH(R'')-OH and A, R' and R'' have the abovementioned meanings, is reacted with an amine of the above formula (4) in which A and $R^1$ have the abovementioned meanings, to form a compound (1) in which both A as well as R', R'' and one of the $R^1$ have the abovementioned meanings and the other $R^1$ represents a radical of the formula (2), or

f) an amine of the above formula (3) in which A, $R^3$, R' and R'' have the abovementioned meanings, is reacted with an amine of the general formula (4) in which A has the abovementioned meaning and $R^1$ is a hydrogen atom, to form a compound (1) in which both A as well as R', R'' and one of the $R^1$ have the herein mentioned meanings and the other $R^1$ is a radical of the formula (2), or

g) a compound of the formula (4) in which A has the abovementioned meaning and $R^1$ is a hydrogen atom, is reacted with a compound of the formula (5) in which R' and R'' have the abovementioned meanings, to form a compound of the above-defined formulae (6a) or (6b) in which both A as well as R' and R'' have the abovementioned meanings, or

h) a compound of the above formula (3) in which $R^3$ is a group of the formula -CH(R')-CH(R'')-OH and A, R' and R'' have the abovementioned meanings, is reacted with a compound of the formula (4) in which A has the abovementioned meaning and $R^1$ is a hydrogen atom, to form a compound of the above-defined formula (6a) or (6b),

characterized by that the reaction of the compounds is carried out in the presence of phosphoric acid or of phosphorous acid in an amount of at least 0.01 mole per mole compound (3) or (5) and at a temperature between 150 and 225°C.

2. A process according to claim 1, characterized by that the reaction is carried out at a temperature between 170 and 200°C.

3. A process according to claim 1 or 2, characterized by that the reaction is carried out in the presence of an amount of 0.01 to 0.3 mole of phosphoric acid or phosphorous acid per mole of the compound of the formula (3) or (5).

4. A process according to claim 1, 2 or 3, in which a compound of the general formula (3c) is reacted with an amine of the general formula (4a)

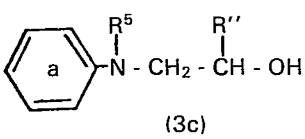

(3c)

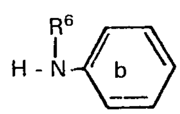

(4a)

in which the benzene nuclei a and b, as substituted or unsubstituted phenyl radicals, can be identical to one another or different from one another, R'' represents a hydrogen atom or the methyl or ethyl group, $R^5$ and $R^6$ are identical to or different from one another and $R^5$ is an optionally substituted alkyl, aralkyl, aryl or cycloalkyl radical, to give a compound of the general formula (7)

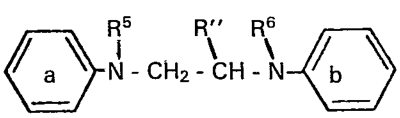

(7)

in which the benzene nuclei a and b and R'', $R^5$ and $R^6$ have the abovementioned meanings.

5. A process according to claim 1, 2 or 3, in which a compound of the general formula (5) is reacted with an amine of the general formula (4a)

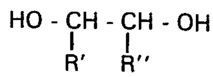

(5)

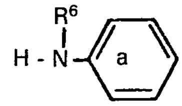

(4a)

in which the benzene nucleus a denotes a substituted or unsubstituted phenyl radical, $R^6$ is an optionally substituted alkyl, aralkyl, aryl or cycloalkyl radical and R' and R'' have the meanings given in claim 1, to form a compound of the general formula (8)

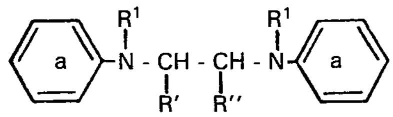

(8)

in which the benzene nuclei a, as substituted or unsubstituted phenyl radicals, are identical to one another and the radicals $R^1$ are each identical to one another and each is an optionally substituted alkyl, aralkyl, aryl or cycloalkyl radical.

6. A process according to claim 1, 2 or 3, in which a compound of the general formula

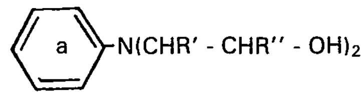

is reacted with a compound of the general formula

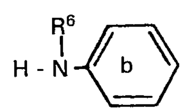

in which the benzene nuclei a and b and the radical $R^6$ have the meaning given in claim 5, but $R^6$ compulsorily is not hydrogen, and R' and R'' have the meanings given in claim 1, preferably both, but especially R', representing hydrogen, to form a compound of the general formula

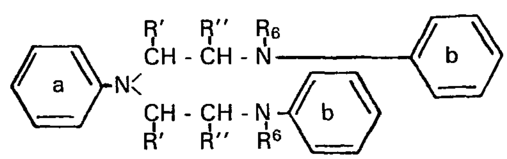

in which a, b, R', R'' and $R^6$ have the meanings just given.

7. A process according to claim 1, 2 or 3, in which a compound of the general formula

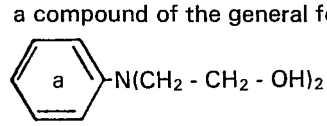

is reacted with a compound of the general formula

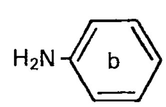

in which the benzene nuclei a and b, as substituted or unsubstituted phenyl radicals, can be identical to one another or different from one another, to form a compound of the general formula

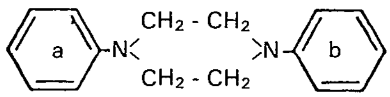

in which a and b have the meaning given.